# EUROPEAN PATENT SPECIFICATION

(11) **EP 4 326 299 B1**
(45) Date of publication and mention of the grant of the patent: **02.12.2026**
(21) Application number: 22720783.4
(22) Date of filing: 21.04.2022
(51) Int. Cl.: A61K 35/76, A61P 1/00, A61P 31/00, A61K 35/741, A61K 35/742, A61K 35/744, A61K 35/745, A61K 35/747, A61K 39/00, G01N 33/50

(54) **BACTERIOPHAGE ECML-123-2 THERAPY AGAINST ADHERENT-INVASIVE ESCHERICHIA COLI**
BAKTERIOPHAGE ECML-123-2 ZUR THERAPIE GEGEN ADHÄRENT-INVASIVE ESCHERICHIA COLI
BACTÉRIOPHAGE ECML-123-2 POUR LE TRAITEMENT DES SOUCHES D'ESCHERICHIA COLI ADHÉRENTES ET INVASIVES

(30) Priority: 22.04.2021 US 202163178119 P; 27.07.2021 US 202163203560 P
(43) Date of publication of application: 28.02.2024
(73) Proprietor: Ferring B.V., 2132 JX Hoofddorp (NL)
(72) Inventor: WANNERBERGER, Kristin, 2132 JX Hoofddorp (NL); SULAKVELIDZE, Alexander, Columbia, Maryland 21046 (US)
(74) Representative: Hoffmann Eitle
(86) International application number: PCT/IB2022/053744
(87) International publication number: WO 2022/224193

(56) References cited:
- WO-A1-2014/177622
- WO-A1-2020/178636
- WO-A2-2019/048930
- WO-A2-2019/157231
- US-B2- 7 625 556
- MATTHIEU GALTIER ET AL: "Bacteriophages targeting adherent invasive Escherichia coli strains as a promising new treatment for Crohn's disease", JOURNAL OF CROHN'S AND COLITIS, 27 January 2017 (2017-01-27), NL, pages jjw224, XP055558846, ISSN: 1873-9946, DOI: 10.1093/ecco-jcc/jjw224
- JOÃO SABINO ET AL: "Review article: bacteriophages in gastroenterology-from biology to clinical applications", ALIMENTARY PHARMACOLOGY & THERAPEUTICS, BLACKWELL SCIENTIFIC PUBLICATIONS LTD., CAMBRIDGE, GB, vol. 51, no. 1, 7 November 2019 (2019-11-07), pages 53 - 63, XP071546599, ISSN: 0269-2813, DOI: 10.1111/APT.15557
- GOGOKHIA LASHA ET AL: "Expansion of Bacteriophages Is Linked to Aggravated Intestinal Inflammation and Colitis", CELL HOST & MICROBE, vol. 25, no. 2, 13 February 2019 (2019-02-13), pages 285, XP085602358, ISSN: 1931-3128, DOI: 10.1016/J.CHOM.2019.01.008

## Description

### FIELD OF THE INVENTION

Described herein are bacteriophage strains and compositions comprising them that effectively target (*e.g.,* infect and lyse) adherent-invasive *Escherichia coli* (AIEC). The bacteriophages are useful, for example, for the prophylactic or therapeutic treatment of subjects infected with AIEC or at risk of infection by AIEC, or in the prophylactic or therapeutic treatment of diseases and conditions associate with AIEC, including inflammatory bowel disease (IBD), and for other uses described herein.

### BACKGROUND

Bacteriophages are viruses that infect bacteria and lyse them as part of their replication/lytic cycle. Bacteriophages (or "phages" for short) derive their name from the Greek word "phago" meaning "to eat" or "bacteria eaters," and were discovered by Felix D'Herelle in the first part of the twentieth century. Phages are specific for their targeted bacterial hosts, and do not infect human or other eukaryotic cells. Bacteriophages have been used therapeutically in humans since 1919 to target pathogenic bacteria. Early enthusiasm led to their use as both prophylaxis and therapy for diseases caused by bacteria. In the U.S. during the 1940s, Eli Lilly commercially manufactured six phage products for human use including preparations targeted against *staphylococci, streptococci* and other respiratory pathogens. With the advent of antibiotics, the therapeutic use of phages gradually fell out of favor in the U.S. and Western Europe and little research was conducted. However, bacteriophage therapy continued to be utilized in Eastern Europe.

Inflammatory bowel disease (IBD) is a category of inflammatory diseases that primarily affect the colon and small intestine. While the etiology of IBD is still being studied, IBD is considered to be an autoimmune disease with genetic, environmental, and microbial contributing factors. The most common forms of IBD are ulcerative colitis (UC) and Crohn's disease (CD). UC is a chronic inflammatory disease of the colon that resembles an infectious disease in its acute stages. UC causes inflammations of the mucosa of the colon, with extension to the submucosa in severe cases. Typically, not only the colon, but also the rectum is attacked, but only rarely is the ileum involved. Ulcer formation and its extent vary with the stage of the disease, but can often be determined macroscopically (such as by sigmoidoscopy or colonoscopy). CD, also known as regional enteritis or colitis granulomatosa, is an inflammatory disease of the intestines that may affect any part of the gastrointestinal tract from mouth to anus, causing a wide variety of symptoms. It primarily causes one or more of abdominal pain, diarrhea, vomiting, and weight loss, but may also cause complications outside the gastrointestinal tract, such as skin rashes, arthritis, inflammation of the eye, tiredness, and lack of concentration. CD is most frequently located in the small intestine (small bowel), especially in the ileum, but may also affect the jejunum and any part of the colon, including the rectum. In the latter case, it may be difficult to differentiate between UC and CD. Generally, the inflammation of CD differs from that of UC by progressing to layers deeper than the mucosa and affecting the epithelium to a lesser degree.

Although the exact causes of IBD are still unknown, a combination of environmental factors and genetic predisposition seem to be involved. For example, CD is thought to be an autoimmune disease in which the body's immune system attacks the gastrointestinal tract, causing inflammation. In patients with CD, abnormal expression of carcinoembryonic antigen-related cell adhesion molecule 6 (CEACAM6) is observed at the apical surface of the ileal epithelium. Additionally, CD ileal lesions and UC ulcers may be colonized by pathogenic adherent-invasive *Escherichia coli* (AIEC). Thus, AEIC is understood to be a microbial factor of pathogenesis in these diseases. *See, e.g.,* Astley, et. al., Eur. J. Clin. Microbiol. Inf. Dis. (2021) 40:181-92.

Both UC and CD are becoming increasingly frequent, especially in developed countries. Neither IBD in general nor UC or CD in particular can be effectively treated with antibiotics (including antibiotics targeting pathogenic *E. coli*)*.* Thus, there remains a need for effective treatments for IBD, including UC and CD.

AIEC infection also is associated with other diseases and conditions, including colon cancer, urinary tract infections, neonatal meningitis, and respiratory diseases and conditions. *See, e.g.,* Astley, *supra* (colon cancer and UTIs); Nash et. al., BMC Genomics (2010) 11:667 (UTIs and neonatal meningitis); Wypych et. al., Nature Immunol. (2010) 20: 1279-90 (lung infections and respiratory diseases and conditions including asthma); Raftery, et. al., Frontiers in Immunol. (2020) 11: Article 2144 (chronic obstructive pulmonary disease). Thus, there remains a need for treatments effective against AEIC for use in treating diseases and conditions associated with AEIC.

WO 2019/157231 A2 relates to a bacteriophage for treatment and prevention of bacteria-associated cancers. WO 2014/177622 A1 relates to bacteriophage therapy. Galtier et. al., J Crohns Colitis. 2017;11(7):840-847 relates to bacteriophages targeting adherent invasive Escherichia coli strains as a treatment for Crohn's disease. WO 2020/178636 A1 relates to a bacteriophage for modulating inflammatory bowel disease.

### SUMMARY OF THE INVENTION

The present invention provides bacteriophage strains that target (*e.g*., infect and lyse) adherent-invasive *Escherichia coli* (AIEC) and compositions comprising one or more different such strains (*e.g*., a "cocktail" of bacteriophage strains) that infect and lyse AIEC as defined in the appended claims. The compositions can be over-the-counter (OTC) or pharmaceutical compositions. The bacteriophages and compositions comprising them are useful for reducing the risk of, preventing, or treating AIEC infection, and for treating diseases and conditions associated with AEIC, including IBD, including CD and UC, colon cancer, urinary tract infections (UTIs), neonatal meningitis, and respiratory diseases and conditions (including asthma, chronic obstructive pulmonary disease (COPD), bronchitis, pneumonia, and lung cancer), and for other methods and uses described herein.

Thus, there are provided compositions comprising a bacteriophage strain capable of producing a lytic infection in an adherent-invasive *Escherichia coli* strain, and a pharmaceutically acceptable carrier, wherein the bacteriophage strain is strain ECML-123-2, or a variant strain thereof, wherein the variant strain has at least 99% average nucleotide identity across its genome (gANI) to said bacteriophage strain ECML-123-2, as defined in the appended claims. Strain ECML-123-2 is deposited with the ATCC under Accession Number PTA-121408. The composition may further comprise one or more bacteriophage strains selected from: strain P2 deposited with the French National Collection of Microorganisms at the Institut Pasteur under Accession Number CNCM I-4695 or a variant strain thereof, wherein the variant strain has at least 80% gANI to said bacteriophage strain P2; strain P8 deposited with the French National Collection of Microorganisms at the Institut Pasteur under Accession Number CNCM I-4700 or a variant strain thereof, wherein the variant strain has at least 80% gANI to said bacteriophage strain P8; strain CLB_P2 deposited with the French National Collection of Microorganisms at the Institut Pasteur under Accession Number CNCM I-4675 or a variant strain thereof, wherein the variant strain has at least 80% gANI to said bacteriophage strain CLB_P2; strain ECML-119 deposited with the ATCC under Accession Number PTA-8351 or a variant strain thereof, wherein the variant strain has at least 80% gANI to said bacteriophage strain ECML-119; strain ECML-363 deposited with the ATCC under Accession Number PTA-121407 or a variant strain thereof, wherein the variant strain has at least 80% gANI to said bacteriophage strain ECML-363; and strain ECML-359 deposited with the ATCC under Accession Number PTA-121406 or a variant thereof, wherein the variant has at least 80% gANI to said bacteriophage strain ECML-359.

In some embodiments, the bacteriophage strains present in the composition comprise or consist of strain ECML-123-2, or a variant strain thereof, wherein the variant strain has at least 99% gANI to said bacteriophage strain ECML-123-2; strain P2, or a variant strain thereof, wherein the variant strain has at least 90% gANI to said bacteriophage strain P2; strain P8, or a variant strain thereof, wherein the variant strain has at least 90% gANI to said bacteriophage strain P8; strain CLB_P2, or a variant strain thereof, wherein the variant strain has at least 90% gaNI to said bacteriophage strain CLB_P2; strain ECML-119, or a variant strain thereof, wherein the variant strain has at least 90% gANI to said bacteriophage strain ECML-119; strain ECML-363, or a variant strain thereof, wherein the variant strain has at least 90% gANI to said bacteriophage strain ECML-363; and strain ECML-359, or a variant thereof, wherein the variant has at least 90% gANI to said bacteriophage strain ECML-359.

In some embodiments, the bacteriophage strains present in the composition comprise or consist of: strain ECML-123-2, or a variant strain thereof, wherein the variant strain has at least 99% gANI to said bacteriophage strain ECML-123-2; strain P2, or a variant strain thereof, wherein the variant strain has at least 95% gANI to said bacteriophage strain P2; strain P8, or a variant strain thereof, wherein the variant strain has at least 95% gANI to said bacteriophage strain P8; strain CLB_P2, or a variant strain thereof, wherein the variant strain has at least 95% gANI to said bacteriophage strain CLB_P2; strain ECML-119, or a variant strain thereof, wherein the variant strain has at least 95% gANI to said bacteriophage strain ECML-119; strain ECML-363, or a variant strain thereof, wherein the variant strain has at least 95% gANI to said bacteriophage strain ECML-363; and strain ECML-359, or a variant thereof, wherein the variant has at least 95% gANI to said bacteriophage strain ECML-359.

In some embodiments, the bacteriophage strains present in the composition comprise or consist of bacteriophages of each of strains ECML-123-2, P2, P8, CLB_P2, ECML-119, ECML-363, and ECML-359.

In any embodiments, the composition optionally further comprises one or more of a probiotic bacteria and a probiotic yeast. The probiotic bacteria may be selected from one or more of *L. acidophilus, L. rhamnosus, L. gasseri, L. reuteri, L. bulgaricus, L. plantarum, L. johnsonii, L. paracasei, L. casei, L. salivarius, L. lactis, B. bifidum, B. longum, B. breve, B. infantis*, *B. lactis, B. adolescentis*, *Streptococcus thermophilus, Bacillus cerus, Bacillus subtilis,* and any combinations thereof. The probiotic yeast may be selected from one or more of *Saccharomyces cerevisiae, Saccharomyces boulardii, Saccharomyces cerevisiae var. boulardii, Issatchenkia occidentalis, Lachancea thermotolerans, Metschnikowia ziziphicola, Torulaspora delbrueckii,* and any combination thereof.

Any embodiments of the foregoing the compositions may be provided in an oral dosage form, optionally provided with an enteric coating, optionally in a form selected from tablets, hard gel capsules, soft gel capsules, dragees, powders, granules, solutions, suspensions, dispersions, syrups, and microgels. Any such composition optionally may further comprise a gastric acid reducing agent, optionally selected from a gastric acid neutralizing agent and a proton pump inhibitor.

Alternatively, any embodiments of the foregoing the compositions may be provided in a dosage form selected from: a rectal dosage form, optionally in a suppository, enema, rectal foam, lotion, or gel; a vaginal dosage form, optionally in a suppository, cream vaginal foam, lotion, or gel; a pulmonary dosage form, optionally in a powder, aerosol, nebulizer, or insufflator composition; and an injectable dosage form.

Any embodiments of the foregoing may be a pharmaceutical composition or an over-the-counter composition. Any embodiments of the foregoing may be a dietary supplement, nutraceutical composition, nutritional supplement, or probiotic composition.

Also provided are bacteriophage compositions as described herein for use in reducing the risk of, or preventing, or treating AIEC infection in a subject in need thereof, or treating inflammatory bowel disease (IBD) as defined in the appended claims.

In accordance with any such composition for use the subject may be at risk of colonization or infection by AIEC or may be colonized or infected by AIEC. In some embodiments, the AIEC is present in one or more of the small intestine or large intestine of the subject. In some embodiments, the AIEC is present in the colon of the subject. In some embodiments, the AIEC is present in the urinary tract of the subject. In some embodiments, the AIEC is present in the lungs the subject. In some embodiments, the AIEC is of one or more strains selected from LF82, 07081, 07082, 07076 and 06075, optionally wherein the AIEC is or comprises strain LF82.

In some embodiments, the composition for use is effective to reduce or prevent AIEC colonization in one or more of the small intestine, large intestine, colon, urinary tract, brain and lungs of the subject.

In some embodiments, the subject is suffering from an inflammatory bowel disease (IBD), optionally wherein the subject is suffering from Crohn's disease (CD), or ulcerative colitis (UC), or recurrence of ileal lesions after surgery, optionally after surgery to remove at least a part of the small intestine in a CD patient.

In some embodiments, the bacteriophage composition is administered orally. Such embodiments may optionally further comprise orally administering to the subject a gastric acid reducing agent, optionally selected from a gastric acid neutralizing agent and a proton pump inhibitor. In other embodiments, the bacteriophage composition is administered by a route selected from rectally, vaginally, pulmonary, and injection.

In any embodiments, the method or use may further comprise administering a probiotic to the subject. Optionally, the probiotic may be provided in the same composition as the bacteriophage, such as when the bacteriophage is administered in an oral composition.

In any embodiments, the subject may additionally be treated with fecal microbiota transplantation.

Also provided is an isolated bacteriophage of strain ECML-123-2 as defined in the appended claims, and isolated variant strains thereof having a gANI of at least 99% to said bacteriophage strain ECML-123-2, optionally wherein the variant strain is a progeny of said bacteriophage strain ECML-123-2, as well as isolated variant strains thereof having a gANI of ≥ 99.9% relative to said bacteriophage strain ECML-123-2. Strain ECML-123-2 is deposited with the ATCC under Accession Number PTA-121408. Also provided in the present disclosure is an isolated progeny strain of any of such bacteriophage strains, which progeny strain has an RFLP DNA profile substantially equivalent to that of said bacteriophage strain ECML-123-2.

Also provided in the present disclosure are compositions comprising a lytic enzyme produced by bacteriophage strain ECML-123-2, or the foregoing variants or progeny thereof.

Also provided in the present disclosure are compositions comprising a derivative product of bacteriophage strain ECML-123-2, or the foregoing variants or progeny thereof, wherein the derivative product has activity against AIEC or encodes a product that has activity against AIEC, optionally wherein the derivative product is one or more selected from DNA, cDNA, mRNA and synthetic polynucleotide sequences, DNA/RNA hybrids, and anti-sigma factor genes and expression products thereof.

Also provided in the present disclosure are vaccines comprising an AIEC bacterial lysate obtained by lysing an AIEC strain with bacteriophage strain ECML-123-2, or the foregoing variants or progeny thereof, or a lytic enzyme of any thereof. Also provided in the present disclosure are AIEC bacterial lysates obtained by lysing an AIEC strain with bacteriophage strain ECML-123-2, or the foregoing variants or progeny thereof, or a lytic enzyme of any thereof, for use in vaccinating a subject against AIEC infection. Also provided in the present disclosure are uses of an AIEC bacterial lysate in the preparation of a medicament for vaccinating a subject against AIEC infection, wherein the AIEC bacterial lysate is obtained by lysing an AIEC strain with bacteriophage strain ECML-123-2, or the foregoing variants or progeny thereof, or a lytic enzyme of any thereof. Also provided in the present disclosure are methods for vaccinating a subject against AIEC infection, comprising administering to the subject a bacterial lysate obtained by lysing an AIEC strain with bacteriophage strain ECML-123-2, or the foregoing variants or progeny thereof, or a lytic enzyme of any thereof.

Also provided in the present disclosure are methods for detecting AIEC bacteria in a sample, comprising treating the sample with bacteriophage strain ECML-123-2, or the foregoing variants or progeny thereof, or a lytic enzyme of any thereof, thereby specifically inducing release of a measurable AIEC bacterial product, and measuring the released AIEC bacterial product. In some aspects, the released AIEC bacterial product is one or more of adenosine triphosphate (ATP) and protein kinase (AKT). In some aspects, the sample is a fecal sample obtained from a subject.

### BRIEF DESCRIPTION OF THE DRAWINGS

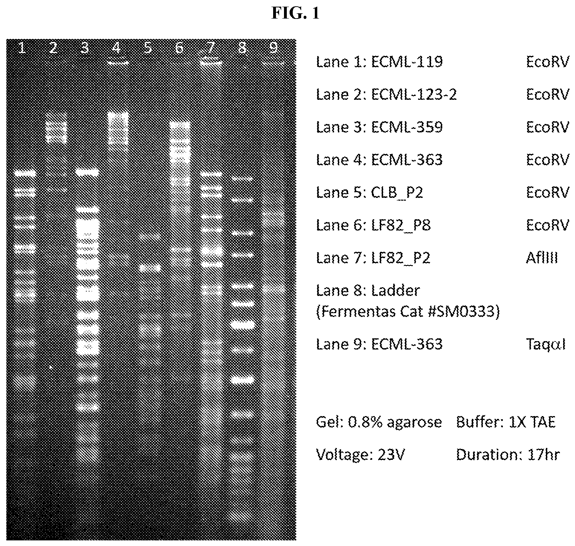

FIG. 1 shows reference DNA RFLP profiles of the bacteriophages described herein.

### DETAILED DESCRIPTION OF THE INVENTION

As noted above, described herein are bacteriophage strains that target (*e.g.*, infect and lyse) AIEC and compositions comprising one or more different such strains that infect and lyse AIEC. The compositions can be over-the-counter (OTC) or pharmaceutical compositions. The bacteriophages and compositions comprising them are useful for reducing the risk of, preventing, or treating AIEC infection, and for treating diseases and conditions associated with AEIC, including IBD, including CD and UC, colon cancer, urinary tract infections (UTIs), neonatal meningitis, and respiratory diseases and conditions (including asthma, chronic obstructive pulmonary disease (COPD), bronchitis, pneumonia, and lung cancer), and for other methods and uses described herein. Without being bound by theory, it is understood that therapy using the bacteriophages and compositions described herein are safe and effective through specific and targeted prevention or reduction of AIEC infection, and thereby are effective against AIEC infection, while maintaining a healthy microbiome by specifically targeting AIEC and avoiding side effects associated with broad-spectrum antibiotic therapies. As illustrated in the examples below, each of the seven phages described herein are individually and collectively effective against a number of different AIEC strains, while at the same time being specific for adherent invasive *E*. *coli* and not lysing common bacteria of the healthy human microbiome. Thus, in contrast to the indiscriminate activity of antibiotics, the phages described herein can be used to target AIEC while having a minimal, if any, effect on the healthy human microbiome.

### Definitions

Technical and scientific terms used herein have the meanings commonly understood by one of ordinary skill in the art to which the present invention pertains, unless otherwise defined. Any suitable materials and/or methods known to those of ordinary skill in the art can be utilized in carrying out the present invention in view of the guidance provided herein; however, specific materials and methods are described for illustrative purposes. Materials, reagents and the like to which reference is made in the following description and examples are obtainable from commercial sources, unless otherwise noted.

As used herein, the singular forms "a," "an," and "the" designate both the singular and the plural, unless expressly stated to designate the singular only.

As used herein, "about" when used with a numerical value means the numerical value stated as well as plus or minus 10% of the numerical value. For example, "about 10" should be understood as both "10" and "9-11."

As used herein, a phrase in the form "A/B" or in the form "A and/or B" means (A), (B), or (A and B); a phrase in the form "at least one of A, B, and C" means (A), (B), (C), (A and B), (A and C), (B and C), or (A, B, and C).

As used herein, the term "comprising" means that the described compositions, methods, or kits include at least the stated elements, and may include other elements that are not specified.

An "adherent-invasive *Escherichia* coli (AIEC) strain" as used herein refers to an *E. coli* strain having a mean invasion potential of equal to or greater than 0.1% in a cell culture of the intestinal cell line I-407, when tested in accordance with the assay described below. *See also* Darfeuille-Michaud et al. Gastroenterology 127: 412-421 (2004). In other words, an AIEC strain has the ability to invade an intestinal cell culture of I-407 with an invasion index equal or superior to 0.1% of the original inoculum (which is deemed to be 100%), when tested in accordance with the invasion assay described below.

Non-limiting examples of AIEC strains are strain LF82 deposited by Université d'Auvergne with the French National Collection at Institut Pasteur under Accession Number CNCM I-4723, LF82S (a streptomycin-resistant, genetically modified variant of strain LF82), and LF82SK (a streptomycin- and kanamycin-resistant, genetically modified variant of strain LF82), both discussed in Galtier et al. J Crohns Colitis. 11(7): 840-47 (2017); strains LF31, LF71, LF123, LF138, LF9, LF15, LF28, LF50, LF65, LF119, LF128, LF130, LF73, LF100, LF110, LF134, LF105, LF49-2, LB11, and LF45-*2 (see, e.g.,* Darfeuille-Michaud et al. Gastroenterology 127:412-421 (2004)), and those discussed in Example 1 below.

The term AIEC "colonization" is used herein to refer to the presence of AIEC in a subject that is not necessarily causing disease in the subject.

The term AIEC "infection" is used herein to refer to AIEC invasion of a subject's tissue that is causing disease in the subject. "Preventing" as used herein means to reduce the risk of AIEC colonization or infection or prevent AIEC colonization or infection, or to reduce the level of AIEC colonization or infection, in a subject in need thereof.

"Treating" as used herein means to reduce the level of AIEC colonization or infection in a subject in need thereof, including to reduce the AIEC colonization or infection to undetectable levels. Such treatment may result in decreasing one or more symptoms characteristic of the disease or condition at issue (if any), such as IBD (such as CD or UC), colon cancer, UTI, neonatal meningitis, or respiratory condition, decreasing the rate of progression of the disease or condition, promoting recovery from the disease or condition, curing the disease or condition, maintaining remission of the disease or condition, and prophylaxis such as prevention of relapse of the disease or condition.

As used herein, the phrases "effective amount" and "therapeutically effective amount" used with reference to a therapeutically active agent (*e.g.*, a phage) means an amount that provides the specific pharmacological effect for which the agent is administered to a subject in need of such treatment, *e.g*., to reduce the risk of, prevent, or reduce AIEC infection. It is emphasized that a therapeutically effective amount will not always be effective to prevent or reduce AIEC infection in a given subject, even though such amount is deemed to be a therapeutically effective amount by those of skill in the art. The therapeutically effective amount may vary based on the specific active agent, route of administration and dosage form, age and weight of the subject, and/or the subject's condition, including the type and severity of the AIEC infection.

The terms "individual," "subject," and "patient" are used interchangeably herein, and refer to any individual mammalian subject, including human subjects, including male and female human subjects. The subject may be suffering from and/or diagnosed with a disease or condition associated with AIEC, such as IBD, colon cancer, UTI, neonatal meningitis, and respiratory diseases or conditions.

Non-limiting examples of IBD include CD, UC, and other chronic inflammatory bowel diseases (chronic IBD) such as microscopic colitis, celiac disease and vasculitis. Thus, the IBD may be CD or UC. In specific embodiments, the IBD is recurrence of ileal lesions after surgery (such as after surgery for the removal of at least a part of the small intestine in CD patients). Recurrence can be measured by the Rutgeerts score. *See, e.g.,* Chongthammakun, et al. Gastroenterol Rep (Oxf) 5: 271-76 (2017).

In some embodiments, the IBD is not caused by a bacterial infection, although a bacterial infection may be concomitant to the IBD (but not necessarily the causative agent). In some embodiments, the subject is infected with AIEC, but does not suffer from IBD. Indeed, some family members of IBD patients may harbor AIEC without suffering from the disease. Likewise, AIEC strains can be found in subjects neither suffering from IBD nor related to subjects suffering from IBD.

As used herein, a "variant" strain of a bacteriophage strain described herein has at least 80% average nucleotide identity across its genome ("gANI") to a reference bacteriophage strain. gANI is a similarity index between a given pair of genomes. Typically, scholars would identify organisms with a gANI ≥ 95% as belonging to the "Same Species." *See* Olm M., "Are these microbes the 'same'?" microBEnet (2; 2017) (available at microbe.net/2017/02/15/are-these-microbes-the-same/), and Jain *et al.* (Jain et al. Nature Comm. 9(1):5114 (2018). A variant strain having at least 90% gANI to the reference strain is presumed and deemed to have the same phenotypic characteristics as said reference bacteriophage strain. A "variant" strain may be obtained independently of the reference strain, may be a progeny of the reference strain, or may be a recombinant derivative of the reference strain (*e.g*., made recombinantly using the reference strain or one or more or all of its genomic sequences as starting material).

As used herein, "isolated" as used with reference to a bacteriophage means the bacteriophage has been removed from its original environment in which it naturally occurs, *e.g*., where it originally was found. An "isolated" bacteriophage may have been purified from, cultivated separately from, or cultured separately from the environment in which it is naturally located or originally was found.

### Bacteriophage Strains And Cocktails

Provided herein are isolated bacteriophages of novel bacteriophage strain ECML-123-2, which has been deposited with the American Type Culture Collection under Accession Number PTA 121408 on July 25, 2014, and variant strains thereof having at least 80%, at least 90%, or at least 95% gANI to the deposited strain. The sequence of ECML-123-2 is set forth in SEQ ID NO:1. As shown in the examples below, strain ECML-123-2 effectively targets (infects and lyses) AIEC.

Also provided herein are compositions comprising isolated bacteriophages of one or more of the following (seven) bacteriophage strains (which includes strain ECML-123-2) and variants thereof having at least 80%, at least 90%, or at least 95% gANI to the deposited strains, including novel combinations ("cocktails") of two or more thereof:
(i) bacteriophage strain vB_EcoM_LF82_P2 (hereinafter "P2") (previously described in WO 2014/177622 and U.S. Pre-Grant Publication 2016/0143965, granted as U.S. Patent No. 11,040,078, and deposited with the French National Collection of Microorganisms at the Institut Pasteur under Accession Number CNCM I-4695 on November 15, 2012);
(ii) bacteriophage strain vB_EcoM_LF82_P8 (hereinafter "P8") (previously described in WO 2014/177622 and U.S. Pre-Grant Publication 2016/0143965, granted as U.S. Patent No. 11,040,078, and deposited with the French National Collection of Microorganisms at the Institut Pasteur under Accession Number CNCM I-4700 on November 15, 2012);
(iii) bacteriophage strain CLB_P2 (previously described in WO 2014/177622 and U.S. Pre-Grant Publication 2016/0143965, granted as U.S. Patent No. 11,040,078, and deposited with the French National Collection of Microorganisms at the Institut Pasteur under Accession Number CNCM I-4675 on September 27, 2012);
(iv) bacteriophage strain ECML-119 (previously described in U.S. Patent No. 7,625,556 and deposited at the American Type Culture Collection under Accession Number PTA-8351 on April 19, 2007);
(v) bacteriophage strain ECML-359 (previously described in Cieplak et al., Gut Microbes 9(5): 391-99 (2018), and deposited with the American Type Culture Collection under Accession Number PTA 121406 on July 25, 2014);
(vi) bacteriophage strain ECML-363 (previously described in Cieplak et al., Gut Microbes 9(5): 391-99 (2018), and deposited with the American Type Culture Collection under Accession Number PTA 121407 on July 25, 2014); and
(vii) bacteriophage strain ECML-123-2 (deposited with the American Type Culture Collection under Accession Number PTA 121408 on July 25, 2014).

Under current taxonomy, strain P2 belongs to family *Myoviridae* of order *Caudovirales,* in the wV8 bacteriophage family; strain P8 belongs to family *Myoviridae* of order *Caudovirales,* in the RB69 bacteriophage family; strain CLB_P2 belongs to family *Myoviridae* of order *Caudovirales,* in the JS98 bacteriophage family; strain ECML-119 belongs to family *Siphoviridae* of order *Caudovirales,* in the EPS7 bacteriophage family; strains ECML-123-2 and ECML-363 belong to family *Myoviridae* of order *Caudovirales,* in the wV7 bacteriophage family; strain ECML-359 belongs to family *Myoviridae* of order *Caudovirales,* in the SP18 bacteriophage family.

As illustrated in the examples, each of these strains specifically targets (infects and lyses) one or more strains of AIEC, and is useful for reducing the risk of infection, preventing infection, and/or treating infection by one or more strains of AIEC, and for other uses described herein, including treating IBD, such as CD or UC. In particular, as illustrated in the examples, each of these strains specifically targets (infects and lyses) one or more of the following AIEC strains: *Escherichia coli* strains LF82, 07081, 07082, 07076 and 06075.

The deposited strains and variant strains thereof having at least 80%, at least 90%, or at least 95% gANI thereto are referred to collectively in the discussion herein as bacteriophages, phages, or strains "as described herein."

As noted above, as used herein "variants" (or "variant strains") include progeny obtained by cultivating the deposited bacteriophages, as illustrated in the examples below. Restriction Fragment Length Polymorphism (RFLP) can be used to identify bacteriophage strains or their progeny. The progeny strain may have a "substantially equivalent" DNA RFLP profile to the DNA RFLP profile of the original bacteriophage strain as defined in Tenover et al., J. Clin. Microbiol. 33(9): 2233-39 (1995). As reported in Tenover, when genomes of identically propagated organisms are electrophoretically analyzed following restriction enzyme digestion, some variation in RFLP profiles may be seen. Tenover describes a system for interpreting chromosomal DNA RFLP profiles using Pulsed-Field Gel Electrophoresis (PFGE). In particular, Tenover sets forth various categories of genetic and epidemiologic relatedness including for identifying organisms that are "indistinguishable" from or "closely related" to each other. As used herein, a bacteriophage strain is "substantially equivalent" to a reference bacteriophage strain if the RLFP profiles are "indistinguishable" or "closely related" under the criteria of Tenover, *supra.*

Thus, a "progeny" strain may have a DNA RFLP profile substantially equivalent to the DNA RFLP profile of the parent deposited bacteriophage strain. A progeny may have ≥ 80% gANI to the parent deposited bacteriophage. A progeny may have ≥ 90% gANI to the parent deposited bacteriophage. A progeny may have ≥ 95% gANI to the parent deposited bacteriophage. A progeny may have ≥ 98% gANI to the parent deposited bacteriophage. A progeny may have ≥ 99% gANI to the parent deposited bacteriophage.

As noted above, variant strains having at least 90% gANI to a reference bacteriophage strain are presumed and deemed to have the same phenotypic characteristics as the reference bacteriophage strain. Thus, the present disclosure includes compositions comprising variants of the deposited strains having at least 90% gANI to a reference deposited strain and other variants having the same phenotypic characteristics as a deposited strain.

The specific isolated bacteriophage strain or combination of isolated strains described herein used in a given composition as described herein can be selected based on the target strain(s) of AIEC. For example, an isolated bacteriophage strain or combination of two or more isolated strains described herein can be tested *in vitro* against the target strain(s) of AIEC to confirm efficacy against the target strain(s) prior to using the isolated bacteriophage strain(s) in a composition to treat a specific subject or group of subjects. Suitable screening methodologies are known in the art and illustrated in the examples herein. For use in such testing, target strain(s) of AIEC can be obtained from clinical specimens obtained from a specific subject or group of subjects, from the environment of a specific subject or group of subjects, or identified and obtained from other sources.

In some aspects, a bacteriophage composition as described herein comprises one or more isolated bacteriophage strains that collectively specifically target (infect and lyse) one or more AIEC strains selected from *Escherichia coli* strains LF82, 07081, 07082, 07076 and 06075. In some aspects, a bacteriophage composition as described herein comprises one or more isolated bacteriophage strains that specifically target AIEC strain LF82.

In some aspects, a bacteriophage composition as described herein comprises isolated bacteriophage strain ECML-123-2 or a variant thereof having at least 80%, at least 90%, or at least 95% gANI to ECML-123-2. In some aspects, a bacteriophage composition as described herein comprises isolated bacteriophage strain ECML-123-2.

In some aspects, a bacteriophage cocktail as described herein comprises isolated bacteriophages of each of the following strains (i) P2 or a variant thereof having at least 80%, at least 90%, or at least 95% gANI to P2; (ii) P8 or a variant thereof having at least 80%, at least 90%, or at least 95% gANI to P8; (iii) CLB_P2 or a variant thereof having at least 80%, at least 90%, or at least 95% gANI CLB_P2; (iv) ECML-119 or a variant thereof having at least 80%, at least 90%, or at least 95% gANI to ECML-119; (v) ECML-359 or a variant thereof having at least 80%, at least 90%, or at least 95% gANI to ECML-359; (vi) ECML-363 or a variant thereof having at least 80%, at least 90%, or at least 95% gANI to ECML-363; and (vii) ECML-123-2 or a variant thereof having at least 80%, at least 90%, or at least 95% gANI to t ECML-123-2.

In some aspects, a bacteriophage cocktail as described herein comprises isolated bacteriophages of each of strains P2, P8, CLB_P2, ECML-119, ECML-359, ECML-363 and ECML-123-2. As illustrated in the examples below, these seven phages have a broad host/target range with some overlap, which may reduce the risk of emergence of bacteriophage-insensitive mutants (BIMs) in the human gut, or in any other organ or elsewhere where these bacteriophages are exposed to their target AIEC bacteria.

In some aspects, a bacteriophage cocktail as described herein comprises isolated bacteriophages of each of the following strains: (i) ECML-123-2 or a variant thereof having at least 80%, at least 90%, or at least 95% gANI to t ECML-123-2, and (ii) one or more selected from the other bacteriophages described herein, *e.g.*, one or more of P2, P8, CLB_P2, ECML-119, ECML-359, and ECML-363, and variants thereof having at least 80%, at least 90%, or at least 95% gANI to the deposited strain. In some aspects, a cocktail comprises (i) ECML-123-2 and (ii) one or more of P2, P8, CLB_P2, ECML-119, ECML-359, and ECML-363.

In some aspects, a bacteriophage cocktail as described herein comprises (i) isolated bacteriophages of one or more strains selected from P2, P8, CLB_P2, and variants thereof having at least 80%, at least 90%, or at least 95% gANI to the deposited strain and (ii) isolated bacteriophages of one or more strains selected from ECML-359, ECML-363, and variants thereof having at least 80%, at least 90%, or at least 95% gANI to the deposited strain. Such a cocktail may optionally further comprise bacteriophages of strain ECML-123-2 or a variant thereof having at least 80%, at least 90%, or at least 95% gANI to ECML-123-2. In some aspects, a cocktail comprises (i) isolated bacteriophages of one or more strains selected from P2, P8, and CLB_P2, and (ii) isolated bacteriophages of one or more strains selected from ECML-359 and ECML-363. Such a cocktail may optionally further comprise bacteriophages of strain ECML-123-2.

The bacteriophage cocktails described herein may comprise an amount of the one or more or two or more isolated bacteriophage strains effective to prevent AIEC colonization, effective to prevent AIEC infection, effective to treat AIEC colonization or effective to treat AIEC infection, such as from 10⁴ to 10¹¹ CFU of the isolated bacteriophages per dose, such as from 10⁶ to 10¹⁰ CFU of the isolated bacteriophages per dose. A bacteriophage cocktail may be prepared with approximately equal amounts of each of the bacteriophages being used. Alternatively, a bacteriophage cocktail may be prepared with different amounts of the bacteriophages, such as to provide enhanced efficacy against selected "problem" AIEC strains while maintaining overall broad lytic activity against all target AIEC.

### Other Active Components

Also provided are compositions that comprise one or more or two or more isolated bacteriophage strains as described herein and further comprise one or more probiotics. As used herein, a "probiotic" includes probiotic bacteria and probiotic yeast. Suitable probiotic bacteria are known in the art and include *Lactobacillus* species (such as *L. acidophilus, L. rhamnosus, L. gasseri, L. reuteri, L. bulgaricus, L. plantarum, L. johnsonii, L. paracasei, L. casei, L. salivarius,* and *L. lactis*), *Bifidobacterium* species (such as *B. bifidum, B. longum, B. breve, B. infantis*, *B. lactis,* and *B. adolescentis*), and *Streptococcus thermophilus, Bacillus cerus,* and *Bacillus subtilis.* Suitable probiotic yeast are known in the art and include *Saccharomyces cerevisiae, Saccharomyces boulardii, Saccharomyces cerevisiae var. boulardii, Issatchenkia occidentalis, Lachancea thermotolerans, Metschnikowia ziziphicola,* and *Torulaspora delbrueckii.*

Thus, in some aspects, a composition as described herein comprises one or more or two or more isolated bacteriophage strains as described herein and one or more probiotics. In specific aspects, a composition as described herein comprises one or more or two or more isolated bacteriophage strains as described herein and one or more strains of probiotic bacteria. In other specific aspects, a composition as described herein comprises one or more or two or more isolated bacteriophage strains as described herein and one or more strains of probiotic yeast. In yet other specific aspects, a composition as described herein comprises one or more or two or more isolated bacteriophage strains as described herein and one or more strains of probiotic bacteria and one or more strains of probiotic yeast. Without wanting to be bound by theory, it is believed that the positive health impact of the AIEC bacteriophages described herein may be further enhanced when combined with a probiotic.

As one specific example, a composition as described herein may comprise
(A) isolated bacteriophages of each of the following strains: (i) P2 or a variant thereof having at least 80%, at least 90%, or at least 95% gANI to P2; (ii) P8 or a variant thereof having at least 80%, at least 90%, or at least 95% gANI to P8; (iii) CLB_P2 or a variant thereof having at least 80%, at least 90%, or at least 95% gANI CLB_P2; (iv) ECML-119 or a variant thereof having at least 80%, at least 90%, or at least 95% gANI to ECML-119; (v) ECML-359 or a variant thereof having at least 80%, at least 90%, or at least 95% gANI to ECML-359; (vi) ECML-363 or a variant thereof having at least 80%, at least 90%, or at least 95% gANI to ECML-363; and (vii) ECML-123-2 or a variant thereof having at least 80%, at least 90%, or at least 95% gANI to ECML-123-2, and
(B) one or more probiotics. As another example, a probiotic cocktail composition as described herein may comprise (A) each of isolated strains P2, P8, CLB_P2, ECML-119, ECML-359, ECML-363 and ECML-123-2 and (B) one or more probiotics. In any aspects comprising a probiotic, the one or more probiotics may be one or more probiotic bacteria selected from *Lactobacillus* species (such as *L. acidophilus, L. rhamnosus, L. gasseri, L. reuteri, L. bulgaricus, L. plantarum, L. johnsonii, L. paracasei, L. casei, L. salivarius,* and *L. lactis*), *Bifidobacterium* species (such as *B. bifidum, B. longum, B. breve, B. infantis*, *B. lactis,* and *B. adolescentis*), and *Streptococcus thermophilus, Bacillus cerus,* and *Bacillus subtilis*; one or more probiotic yeast selected from *Saccharomyces cerevisiae, Saccharomyces boulardii, Saccharomyces cerevisiae var. boulardii, Issatchenkia occidentalis, Lachancea thermotolerans, Metschnikowia ziziphicola,* and *Torulaspora delbrueckii,* or any combination thereof.

In any aspects, a composition comprising one or more or two or more isolated bacteriophage strains and a probiotic as described herein may be an OTC composition or a pharmaceutical composition. In some aspects, a composition comprising one or more or two or more isolated bacteriophage strains and a probiotic as described herein is formulated for oral administration, as discussed in more detail below.

As set forth above with reference to the bacteriophage cocktails described herein, compositions described herein may comprise an amount of the isolated bacteriophage strains effective to prevent AIEC colonization, effective to prevent AIEC infection, effective to treat AIEC colonization or effective to treat AIEC infection, such as from 10⁴ to 10¹¹ CFU of the isolated bacteriophage strains per dose, such as from 10⁶ to 10¹⁰ CFU of the isolated bacteriophage strains per dose.

Also provided herein are derivative products of a bacteriophage as described herein, and compositions comprising one or more such derivative products. In some aspects, a composition as described herein comprises one or more or two or more bacteriophage strains as described herein and further comprises one or more such derivative products.

A "derivative product" or "phage derivative product" as used herein refers to substances that constitute subunits or expression products of the bacteriophages described herein, including (but not limited to) nucleic acids, partial or complete genes, lytic enzymes and other gene expression products, and other structural components of a bacteriophage, such as polyribonucleotide(s) and polydeoxyribonucleotide(s), including modified or unmodified bacteriophage DNA, cDNA, mRNA and synthetic polynucleotide sequences, as well as DNA/RNA hybrids, such as derivative products that have activity against AIEC or encode products that have activity against AIEC. Thus, one or more derivative products of the bacteriophages described herein may be included in the compositions described herein and used in the methods described herein.

Anti-sigma factor genes and expression products thereof are examples of such derivative products. One or more of the phages described herein may encode an anti-sigma factor gene that inhibits bacterial transcriptional activity of the host (target) AIEC. *See, e g.,* Hughes, et al. Ann. Rev Microbiol 52:231-86 (1998). The anti-sigma factor gene grants such phages an enhanced ability to inhibit transcription within target (AIEC) bacterial cell, thereby enhancing the phage's ability to lyse the target bacterial cell. Thus, anti-sigma factor genes and expression products thereof are examples of derivative products that may be included in the compositions described herein and used in the prophylactic and therapeutic treatment methods described herein, such as to enhance the potency of the composition and/or the efficacy of the method.

Lytic enzymes are another example of phage derivative products described herein. The bacteriophages described herein encode one or more lytic enzymes involved in lysing the host (target) AIEC. Phage lytic enzymes are produced by bacteriophages either as part of their virion to facilitate bacterial infection through local peptidoglycan degradation, or as soluble proteins to induce massive cell lysis at the end of the lytic replication cycle. *See, e.g.,* Briers, Viruses 11(2): 113 (2019). Lytic enzymes can cause rapid lysis of their targeted bacteria, resulting in significant reduction or elimination of the targeted bacterium's levels. In addition to enhancing the potency or efficacy of the compositions and prophylactic and therapeutic treatment methods described herein, lytic enzymes can be used in detection assays for rapid identification of specific bacteria. *See, e.g.,* Nelson, et al., "Using bacteriophage lytic enzymes as a diagnostic tool for rapid identification of specific bacteria." In American Society for Microbiology General Meeting, Salt Lake City, Utah (2002). Lytic enzymes for use in the compositions and methods described herein may be isolated from phage cultures, or prepared by recombinant techniques. Thus, lytic enzymes may be included in the compositions described herein and used in the prophylactic and therapeutic treatment methods and detection methods described herein, such as to enhance the potency of the composition and/or the efficacy of the treatment method, or to detect specific target AIEC bacteria.

Thus, in some aspects, a composition as described herein comprises one or more or two or more bacteriophage strains and one or more such phage derivative products, such as one or more lytic enzymes. In some aspects, a composition as described herein includes one or more lytic enzymes produced by one or more of the bacteriophages formulated in the composition. In some aspects, a composition as described herein additionally or alternatively comprises one or more anti-sigma factor genes or expression products thereof of one or more of the bacteriophages formulated in the composition. In any aspects, a composition comprising one or more or two or more bacteriophage strains and a phage derivative product such as phage lytic enzyme(s) may be an OTC composition or a pharmaceutical composition. Any such composition also may comprise one or more probiotics as discussed above.

### Other Composition Features

As noted above, provided herein are compositions comprising one or more or two or more of the bacteriophage strains described herein. As noted above, the compositions may be OTC compositions or pharmaceutical compositions. For example, the composition may be a pharmaceutical composition, dietary supplement, nutraceutical composition, nutritional supplement, or probiotic composition.

An OTC or pharmaceutical composition as described herein comprises the bacteriophage strain(s) (and, in some aspects, probiotic(s) and/or phage derivative products) and one or more pharmaceutically acceptable carriers, and, optionally one or more pharmaceutically acceptable excipients. Suitable carriers and excipients for bacteriophage(s) are known in the art. Typically, a pharmaceutically acceptable carrier is a pharmaceutically-acceptable, non-toxic carrier, filler, or diluent, suitable for use as a vehicle for formulating pharmaceutical compositions for administration to the target patient (animal or human) by the intended route of administration.

Pharmaceutically acceptable excipients, also referred to as auxiliary agents or accessory ingredients, encompass those conventionally used in pharmaceutical compositions for administration to the target patient (animal or human) by the intended route of administration, such as, but not limited to, matrix-forming agents, thickeners, binders, lubricants, pH adjusting agents, protecting agents, viscosity enhancers, wicking agents, disintegrants, including non-effervescent and effervescent disintegrants, surfactants, antioxidants, wetting agents, colorants, flavoring agents, taste-masking agents, sweeteners, preservatives and so forth. In addition to being pharmaceutically acceptable, the auxiliary agents typically will be selected to be compatible with the other ingredients of the composition, including the bacteriophage(s) and probiotic(s) (if present).

The OTC and pharmaceutical compositions described herein may be formulated for any suitable route of administration (which may depend on the site of AIEC colonization or infection). Phage compositions for different routes of administration have been disclosed. *See, e.g.,* Qadir et al., Brazilian J. Pharm. Sci. (2018) 54(1). For example, phages can be formulated for routes of administration including oral, buccal, sublingual, rectal, nasal, topical, otic, vaginal, bronchial, pulmonary, or parenteral (including subcutaneous, intramuscular, intravenous, intradermal, intraperitoneal, intrapleural, intravesicular and intrathecal) administration, or for administration via an implant, or urinary tract rinse or catheter. The composition or route of administration may be selected to provide a targeted effect of one or more bacteriophages described herein, and may depend on the site(s) of colonization or infection.

The OTC and pharmaceutical compositions may be prepared by any suitable method for making the dosage form at issue, with such methods being well known in the art of pharmacy. Such methods typically will include the step of bringing in association one or more bacteriophages as described herein (and, optionally, one or more probiotics and/or phage derivative products) and a pharmaceutically acceptable carrier, and, optionally, one or more pharmaceutically acceptable auxiliary agents.

In some aspects, a composition as described herein is formulated for oral administration and administered orally. Dosage forms for OTC and pharmaceutical compositions suitable for oral administration are known in the art, and include discrete dosage forms such as tablets (including chewable tablets), hard gel capsules containing a dry phage-containing composition, soft gel capsules containing a liquid phage-containing composition, and dragees, and bulk dosage forms such as powders, granules, solutions, suspensions, dispersions, syrups, microgels, and the like.

Dosage forms of OTC and pharmaceutical compositions formulated for oral administration may be formulated to protect the phages from the acidic environment of the stomach. For example, compositions formulated for oral administration may be provided with or comprise a coating, such as an enteric coating or delayed release coating (such as in the form of coated tablets or capsules, or granules provided with a coating) to protect the bacteriophage (and, optionally the probiotic(s) and/or phage derivative products such as lytic enzyme(s)) and maintain viability during passage through the acidic environment of stomach, or may be microencapsulated (such as in alginate-chitosan microspheres). Suitable coating and microencapsulation materials are known in the art. Additionally or alternatively, compositions formulated for oral administration may be formulated with or administered with an agent that reduces stomach acids (e.g., a gastric acid reducing agent), such as a gastric acid neutralizing agent (e.g., bicarbonate, such as sodium bicarbonate), or a proton pump inhibitor (e.g., omeprazole).

Additionally or alternatively, such compositions may be provided with or comprise a delayed release coating to provide release of viable phages (and, optionally the probiotic(s) and/or phage derivative products such as lytic enzyme(s)) at a desired site of release, such as in the small intestine (such as for treating CD) or colon (such as for treating UC), or in the urinary tract (such as for treating UTI). Additionally or alternatively, such compositions may be provided with or comprise an extended release coating to provide release of the phage (etc.) over an extended period of time. Suitable coating materials for such coatings are known in the art.

For example, a composition as described herein formulated for oral administration may comprise one or more of the following ingredients: water, such as deionized water, pharmaceutical grade water, or mineral water; sodium chloride, sodium bicarbonate; a buffer solution (such as Tris-HCl at pH 7.0-7.5); sweeteners, such as sucrose (e.g., a 5% sucrose solution), trehalose, maltodextrin, glycerol, dextran, and sorbitol, cellulose; thickeners, such as tapioca, dextrin, gellan gum, and gelatin; and other excipients such as hydroxypropyl methylcellulose, poly(acrylic acid) ("PAA"), poly(ethyleneglycol) ("PEG"), and casein, and combinations of any two or more thereof.

In alternative aspects, a composition as described herein is formulated for rectal administration. Dosage forms for OTC and pharmaceutical compositions suitable for rectal administration are known in the art, and may include a suppository, rectal foam, lotion, gel or enema for rectal administration.

In alternative aspects, a composition as described herein is formulated for vaginal administration. Dosage forms for OTC and pharmaceutical compositions suitable for vaginal administration are known in the art, and may include a suppository, cream, vaginal foam, lotion, gel, vaginal rinse, or catheter for vaginal administration.

In alternative aspects, a composition as described herein is formulated for parenteral administration, such as by infusion or injection, including subcutaneous, intramuscular, intravenous, intradermal, intraperitoneal, intrapleural, intravesicular, and intrathecal injection. Compositions suitable for parenteral administration are known in the art, and may include sterile aqueous and non-aqueous compositions formulated for injection or infusion. Such compositions may be provided in unit-dose or multi-dose containers, for example pre-filled syringes, sealed vials, sealed ampoules, or sealed pouches. Such compositions may be ready-to-use, or may be freeze-dried (lyophilized) or spray-dried compositions that are reconstituted prior to use with a sterile liquid carrier, for example, water.

In alternative aspects, a composition as described herein is formulated for topical administration. Dosage forms for OTC and pharmaceutical compositions suitable for topical administration are known in the art, and include solutions, emulsions, creams, lotions, gels, and sprays. For example, the composition as described herein may be formulated as a gel for topical administration.

In alternative aspects, a composition as described herein is formulated for pulmonary or bronchial administration, such as by oral or nasal inhalation. Dosage forms for compositions suitable for pulmonary administration are known in the art, and include powder compositions and fine particle formulations (e.g., dusts or mists) which may be generated by and administered via metered dose pressurized aerosols, nebulizers, insufflators, or the like.

### Treatments

The bacteriophages and compositions described herein can be used in prophylactic and therapeutic treatment methods to reduce the risk of or prevent AIEC colonization or infection or to treat AIEC colonization or infection, or to modulate a subject's microbiome (such as by preventing or reducing colonization by AIEC), or for treating AIEC-related diseases or conditions (such as IBD, including CD and UC, colon cancer, UTI, neonatal meningitis, respiratory diseases and conditions (including asthma, COPD, bronchitis, pneumonia, and lung cancer), etc., or for similar effects.

The target AIEC may be any strain of AIEC, and typically will be a strain of AIEC targeted (infected and lysed) by one or more of the phages present in the composition. In some aspects, the target AIEC is one or more selected from LF82, 07081, 07082, 07076 and 06075. In specific aspects, the target AIEC is or includes LF82.

The bacteriophages and compositions described herein may be used prophylactically or therapeutically to treat any subject in need thereof, including any subject at risk of AIEC infection or any subject infected with AIEC, including subjects suffering from a disease or condition associated with AIEC, including IBD, including CD or UC, colon cancer, UTI, neonatal meningitis, or a respiratory disease or condition, such as asthma, COPD, bronchitis, pneumonia, and lung cancer. The subject may be at risk of colonization with AIEC, or colonized with AIEC. The subject may be at risk of infection by AIEC, or infected with AIEC. In infected subjects, the AIEC may be present in any tissue, including one or more of the small intestine, the large intestine, the ileum, the colon, the urinary tract, the brain, and/or the lungs.

The subject may be suffering from any IBD, including UC or CD, including microscopic colitis, celiac disease, and vasculitis. In specific aspects, the subject is suffering from recurrence of ileal lesions after surgery, such as may occur in a CD patient after surgery removing at least a part of the small intestine. As noted above, such recurrence can be measured by the Rutgeerts score. *See, e.g.,* Chongthammakun, et al. Gastroenterol Rep (Oxf) 5: 271-76 (2017).

Additonally or alternatively, the subject may be suffering from another disease or condition associated with AIEC, such as colon cancer, UTI, neonatal meningitis, and respiratory diseases and conditions such as asthma and COPD.

In accordance with any of the prophylactic and therapeutic treatment methods described herein, the bacteriophages and compositions described herein can be administered according to any effective dosing regimen, which may vary based on the prophylactic or therapeutic effect to be achieved (*e.g*., prevention vs. treatment of colonization or infection or disease), the specific bacteriophages used, the route of administration and dosage form, and patient characteristics such as one or more of the age and weight of the patient, the patient's condition, and the type and severity of the AIEC infection or disease status. Exemplary dosages are described below for illustrative purposes only.

A typical bacteriophage dose for a human patient is likely to contain from 10³ to 10¹² plaque forming units (PFU) of the bacteriophage(s), including from 10⁴ to 10¹¹, such as 10⁶ to 10¹⁰, which may be administered one or more times per day, such as one, two, three, four, or five times per day. The dose may be provided in a single discrete dosage form (e.g., one tablet or capsule) or multiple discrete dosage forms (e.g., two, three, four, or more tablets or capsules), or in a suitable volume of a bulk dosage form (e.g., 1 mL of a liquid composition). The treatment may continue for one or more days, including one day, one week, two weeks, three weeks, one month, or longer.

One example of a suitable dose regimen is 1-3 capsules or tablets, each containing 10⁸-10¹⁰ PFUs bacteriophage(s), orally administered 1-3 times a day for 1-4 weeks. Another example of a suitable dose regimen is 1 mL of a liquid composition containing 10⁸-10¹⁰ PFUs bacteriophage(s), orally administered 1-3 times a day for 1-4 weeks. As illustrated in Example 6, the liquid composition may be diluted in a further liquid composition prior to consumptions, such as in 15-50 mL of a bicarbonate solution. In such aspects, the bicarbonate solution also may function to neutralize stomach acid.

As noted above, the bacteriophage compositions and methods described herein may be used in conjunction with an agent that reduces stomach acids. For example, a subject undergoing treatment may also be treated with a proton pump inhibitor (e.g., omeprazole), and/or may also consume a gastric acid neutralizer, such as bicarbonate (e.g., sodium bicarbonate) shortly before taking a dose of the bacteriophage compositions.

In some aspects relating to the treatment of IBD, the bacteriophage compositions and methods described herein are used in conjunction with fecal microbiota transplantation (FMT). FMT is a therapeutic intervention wherein fecal samples are transferred from healthy donors into patients, in order to recolonize the patient's gut microbiome with colonic bacteria of the healthy donor. FMT can involve transplantation of donor stool samples or of microbes isolated from donor stool samples. Transplantation typically is effected by enema or colonoscopy, but can be effected through the upper GI tract. While FMT currently is primarily used to treat *Clostridium difficile* infection, it also may be useful in treating IBD patients, including CD and UC patients.

### AIEC Detection Methods

The bacteriophages described herein (or lytic enzymes thereof) also can be used in *in vitro* assays to detect target bacteria (e.g. AIEC) in biological samples obtained from a subject, such as to diagnose whether a subject is infected with AIEC and/or to assess the level of infection, including to assess the need for or efficacy of treatment as described herein. Without being bound by theory, it is believed the bacteriophages described herein (or their lytic enzymes) specifically lyse the targeted bacteria (*e.g*., AIEC) without affecting other prokaryotic or eukaryotic cells that may be present, thus specifically inducing release of measurable products of the target bacteria, such as adenosine triphosphate (ATP) and/or protein kinase (AKT) specific to the target AIEC. Thus, a detection assay can be conducted based on the detection of ATP of the target bacteria.

An illustrative example of such an assay is as follows. Samples of clinical material (e.g., fecal specimens) to be analyzed are obtained and suspended in an appropriate buffer. One or more bacteriophages (or lytic enzymes thereof) are added to the suspension, as a result of which any targeted bacterial cells present in the samples are lysed and their ATP is released. To detect the ATP, a luciferin + luciferase preparation is added, and luminescence is measured, such as by using a luminometer. A quantitative assay may be developed by creating a calibration curve between the luminometer readings and the number of targeted bacteria cells lysed (in general, the average amount of ATP per bacterial cell is 0.5-1.0 fg). Absence of luminescence indicates absence of targeted bacteria cells in the sample analyzed.

### Vaccines and Bacterins

Also provided are vaccines and bacterins prepared using the bacteriophages (or lytic enzymes thereof) described herein. For example, the bacteriophages described herein (including variants of the deposited strains) or lytic enzymes thereof can be used to lyse specific strains of targeted bacteria (e.g., targeted AIEC), to obtain bacterial lysates containing immunological epitopes of the bacteria, which in turn can be used to prepare vaccines/bacterins against the targeted AIEC. The final vaccine/bacterin preparations can be prepared by methods known in the art. When used to obtain the bacterins, the phage may be removed to obtain the final vaccine/bacterin preparation. Alternatively, the phage may be retained in the final preparation, in a viable, active state. In such aspects, the phage as present in the vaccine/bacterin formulation may remain active against the targeted bacteria (e.g., targeted AIEC), providing another mechanism of action of efficacy of the preparation, e.g., it may lyse target bacteria (including AIEC) present in the vaccinated subject. For example, the final vaccine/bacterin preparation may be prepared to have the phage present at levels ranging from 10³-10¹² PFU/ml. For example, the final vaccine/bacterin preparation may be prepared to have the phage present at levels ranging from 10⁶-10¹⁰ PFU/ml.

In specific aspects, a vaccine/bacterin preparation is prepared against prevalent, problematic strains of the targeted bacteria to obtain vaccine/bacterin preparations containing immunological epitopes that are most relevant for protecting a target patient population against infection. In further specific aspects, the bacteriophage is retained unaltered in the final vaccine/bacterin preparation as discussed above.

Bacteriophage-based vaccines and bacterins also may be prepared by using recombinant constructs expressing relevant genes of the bacteriophage (e.g., genes associated with lytic activity) or using isolated or recombinantly produced lytic enzymes, which can be used in place of phages *per* se in a protocol as outlined above. An example of this general methodology is outlined in Panthel et al., Infect Immun.71(1):109-16 (2003).

In accordance with any of these aspects, also provided are uses of vaccines comprising bacterins as described above to immunize a subject in need thereof, such as a subject suffering from or at risk of infection by AIEC, or a subject at risk of developing or suffering from IBD. Suitable vaccination protocols can be developed by those skilled in the art based on the guidance provided herein.

### Kits

Also provided are kits for practicing the various aspects described herein.

A kit for prophylactic or therapeutic uses of the bacteriophage(s) described herein may comprise a composition as described herein together with instructions for the prophylactic or therapeutic use of the composition as herein described, optionally together with packaging material.

A kit for prophylactic or therapeutic uses of the vaccine(s)/bacterin(s) may comprise a vaccine/bacterin as described herein together with instructions for the prophylactic or therapeutic use of the vaccine/bacterin as herein described, optionally together with packaging material.

A kit for an AIEC detection assay may comprise one or more bacteriophages as described herein (or one or more lytic enzymes thereof) together with instructions for the use of the bacteriophage(s) (or enzymes) in an AIEC detection assay as herein described, optionally together with packaging material.

### EXAMPLES

The invention is further described in the following examples, which are not in any way intended to limit the scope of the invention.

### EXAMPLE 1: ISOLATION OF AIEC STRAINS

### AIEC Invasion Assay

AIEC can be identified by an invasion assay using Intestine-407 cells (I-407 cells) obtained from the Intestine-407 (I-407) cell line, which is an intestinal cell line used as a model of undifferentiated intestinal epithelial cells, derived from human embryonic jejunum and ileum commercially available from Flow Laboratories Inc. (Mc Lean, VA).

For example, I-407 cells are seeded in 24-well tissue culture plates (Polylabo, Strasbourg, France) at a density of 4,105 cells/well and incubated for 20 hours. The cell monolayers are washed twice with PBS (pH 7.2). Bacterial invasion of epithelial cells is measured using the gentamicin protection assay (Falkow et al. Rev. Infect. Dis. 9 (Supp. 5): S450-55 (1987)). Each monolayer is inoculated in 1 mL of cell culture medium lacking antibiotics with a multiplicity of infection of 10 bacteria per epithelial cell. After a three hour incubation period at 37°C with 5% CO₂, the monolayers are washed three times with PBS. Fresh cell culture medium containing 100 µg/mL of gentamicin (Sigma, St. Louis, MO) is added for 1 hour to kill extracellular bacteria before lysis of the monolayers with 1% Triton X-100 (Sigma) in deionized water. This concentration of Triton X-100 has no effect on bacterial viability for at least 30 minutes. Samples are diluted and plated onto Mueller-Hinton agar plates to determine the number of colony-forming units. Results of *E. coli* invasive ability assessed with the I-407 cell line are expressed as the percentage of intracellular bacteria relative to the initial inoculum.

Using such a protocol, one hundred and sixty-six (166) AIEC strains, including *E. coli* strain LF82, were isolated from fresh feces of CD patients, their family members and control subjects, as described in U.S. Pre-Grant Publication 2016/0143965 (U.S. Patent No. 11,040,078). These AIEC strains were used to assess activity of the phages described herein, as reported in Example 3 below.

### EXAMPLE 2: PHAGE ISOLATION AND CHARACTERIZATION

Phages P2 and P8 were isolated from sewage water as described in WO 2014/177622.

Phage P2 was deposited with the French National Collection of Microorganisms at the Institut Pasteur under the terms of the Budapest Treaty under Accession Number CNCM I-4695 on November 15, 2012. Phage P8 was deposited with the French National Collection of Microorganisms at the Institut Pasteur under the terms of the Budapest Treaty under Accession Number CNCM I-4700 on November 15, 2012.

Phage CLB_P2 was isolated as described in Maura et al. Environmental Microbiology 14(8): 1844-54 (2012). Phage CLB_P2 belongs to the JS98 bacteriophage family, and was deposited with the French National Collection of Microorganisms at the Institut Pasteur under the terms of the Budapest Treaty under Accession Number CNCM I-4675 on September 27, 2012.

Phages ECML-119, ECML-123-2, ECML-359, and ECML-363 were isolated from brackish seawater (ECML-119 and ECML-123-2) or sewage (ECML-359 and ECML-363) by plaque assay methodologies that are generally known in the art. The isolation of ECML-119 is described in U.S. Patent No. 7,625,556. The other strains were isolated by similar methods. In general, bacteriophage preparations are contacted with bacterial lawns of target bacteria (*e.g*., AIEC) and incubated to permit formation of plaques in the bacterial lawns which mark the presence of bacteriophage having lytic specificity for the target bacteria. Plaques are harvested, diluted, and re-plated on bacterial lawns through a process of serial enrichment until a single bacteriophage species (monophage) is obtained, as determined by a stable restriction fragment length profile (RFLP) of the bacteriophage DNA.

Phage ECML-119 was deposited with the with the American Type Culture Collection under Accession Number PTA-8351 on April 19, 2007.

Phage ECML-123-2 was deposited with the American Type Culture Collection under Accession Number PTA 121408 on July 25, 2014. Its sequence is set forth in SEQ ID NO:1.

Phage ECML-359, which belongs to the SP18 bacteriophage family, and was deposited with the American Type Culture Collection under Accession Number PTA 121406 on July 25, 2014.

Phage ECML-363, which belongs to the wV7 bacteriophage family, and was deposited with the American Type Culture Collection under Accession Number PTA 121407 on July 25, 2014.

### Electron Microscopy Of Bacteriophages

Morphology of the phages was examined by transmission electron microscopy of purified phage particles. A high titer (approximately 10⁹ PFU/mL) of phage-containing cell free lysate (pH=7) was applied to the surface of a formvar Carbon Film (200 mesh copper grids). The samples were negatively stained with 1% phophotungstic acid then examined under Hitachi H-7000 transmission electron microscope operated at 100 kV. The phages were classified according to the guidelines of the International Committee on Taxonomy of Viruses (ICTV, 1995) based on their morphological features. Under current taxonomy, bacteriophage P2 belongs to family *Myoviridae* of order *Caudovirales,* in the wV8 bacteriophage family; bacteriophage P8 belongs to family *Myoviridae* of order *Caudovirales,* in the RB69 bacteriophage family; bacteriophage CLB_P2 belongs to family *Myoviridae* of order *Caudovirales,* in the JS98 bacteriophage family; bacteriophage ECML-119 belongs to family *Siphoviridae* of order *Caudovirales,* in the EPS7 bacteriophage family; bacteriophages ECML-123-2
and ECML-363 belong to family *Myoviridae* of order *Caudovirales,* in the wV7 bacteriophage family; bacteriophage ECML-359 belongs to family *Myoviridae* of order *Caudovirales,* in the SP18 bacteriophage family.

Each phage was determined to be a lytic, tailed, double-stranded DNA phage. Phage sequencing did not show the presence of any undesirable toxin or antibiotic resistance encoding genes. In particular, the phage sequences were analyzed against a database of toxin and other undesirable genes (*e.g*., those listed in 40 CFR 725.421) and it was confirmed that none of the seven phages described herein include any of those "undesirable" genes.

### Restriction Fragment Length Polymorphism (RFLP) Profile

Reference DNA RFLP profiles of the bacteriophages described herein are shown in FIG. 1. DNA was isolated from the bacteriophage using Qiagen Plasmid Miniprep or Midiprep kits (Valencia, CA) according to the manufacturer's directions. The DNA was quantitated by measuring absorbance at 260 nm. Approximately 0.5-1.0 µg of DNA was digested with an appropriate restriction enzyme as indicated in the figure, and RFLP profile was determined on 1% agarose gel after staining with ethidium bromide.

### EXAMPLE 3: PHAGE ACTIVITY-IN VITRO SCREENING

The bacteriophages described herein were screened for activity against AIEC in an *in vitro* spot test assay.

The spot test assay was carried out by applying two concentrations of monophage bacteriophage solutions (about 2x10⁴ PFU/mL and about 1x10⁹ PFU/mL) to the surface of a Petri dish covered by one strain of AIEC bacteria. After overnight incubation at 30°C, plaques were examined for zones of lysis, indicating the AIEC tested was susceptible to the bacteriophage. Table 2 shows the result of testing the seven deposited phages described herein against 38 of the 166 AIEC strains identified in U.S. Pre-Grant Publication 2016/0143965 (U.S. Patent No. 11,040,078) (*see* Example 1). In the table below, a result of (++) indicates the AIEC was susceptible to the bacteriophage at both concentrations; a result of (+) indicates the AIEC was susceptible to the bacteriophage at only the highest concentration, and a result of (-) indicates the AIEC was resistant to the bacteriophage.

| **AIEC strain** | **Bacteriophage** | | | | | | |
|---|---|---|---|---|---|---|---|
| | **ECML-119** | **ECML-123-2** | **ECML-359** | **ECML-363** | **CLB-P2** | **LF82-P2** | **LF82_P8** |
| LF82 WT | - | + | - | + | ++ | ++ | ++ |
| 06023 | ++ | - | + | - | + | - | + |
| 06030 | - | - | - | - | ++ | - | + |
| 06033 | - | - | - | - | ++ | + | + |
| 06072 | - | ++ | + | ++ | ++ | ++ | + |
| 06073 | - | ++ | + | ++ | ++ | ++ | ++ |
| 06075 | - | ++ | + | ++ | ++ | ++ | ++ |
| 06088 | - | ++ | - | ++ | - | + | - |
| 06089 | - | ++ | - | ++ | - | + | - |
| 06122 | - | - | - | - | - | - | + |
| 06150 | ++ | - | + | + | + | - | + |
| 06351 | - | - | - | - | + | + | + |
| 06353 | - | + | - | + | + | + | - |
| 06354 | - | + | - | + | + | + | - |
| 06356 | - | - | - | + | + | + | - |
| 06357 | - | + | - | + | + | + | - |
| 06358 | - | + | - | + | + | + | - |
| 06359 | - | + | - | + | + | + | - |
| 06361 | - | - | - | - | + | ++ | + |
| 06362 | - | - | - | - | + | - | - |
| 07045 | + | ++ | + | + | + | - | - |
| 07046 | + | ++ | ++ | + | + | - | + |
| 07048 | - | ++ | + | + | + | - | - |
| 07051 | - | ++ | ++ | ++ | + | - | - |
| 07075 | - | ++ | + | ++ | - | - | - |
| 07076 | + | + | + | - | + | + | + |
| 07077 | - | ++ | + | ++ | - | - | - |
| 07078 | + | + | + | - | ++ | ++ | + |
| 07081 | + | + | + | - | + | + | + |
| 07082 | ++ | ++ | ++ | ++ | ++ | ++ | ++ |
| 07107 | - | + | - | - | ++ | + | + |
| 07126 | - | + | - | - | + | + | + |
| 07127 | - | + | - | - | ++ | + | ++ |
| 07128 | - | + | - | - | ++ | + | + |
| 07134 | ++ | + | + | + | + | - | - |
| 07135 | ++ | + | + | + | + | - | - |
| 07136 | ++ | + | + | + | + | - | - |
| 07137 | ++ | + | ++ | + | + | - | - |

The following table shows the number of AIEC strains targeted (infected and lysed) by each of the seven phages described herein

| **Results / Strains** | **Bacteriophage** | | | | | | |
|---|---|---|---|---|---|---|---|
| | **ECML-119** | **ECML-123-2** | **ECML-359** | **ECML-363** | **CLB_P2** | **LF82_P2** | **LF82_P8** |
| + | 5 | 17 | 15 | 15 | 22 | 16 | **15** |
| ++ | 7 | 12 | 4 | 9 | 11 | 7 | 5 |
| Total / 38 | 12 | 29 | 19 | 24 | 33 | 23 | 20 |

Separately, the lytic range of each of the seven phages was assessed on 210 AIEC strains obtained from European and U.S. collections at two concentrations: 2 x 10⁴ PFU/mL and 1 x 10⁹ PFU/mL. As summarized in the following table, 149 of the 210 isolates (71%) were susceptible at 2 x 10⁴ PFU/mL to at least one of the seven monophages, while 95% were susceptible at the higher phage concentration (1 x 10⁹ PFU/mL), which is the concentration more commonly used in the Spot Test assay.

| **Phage** | **2x10⁴ PFU/mL** | | **1x10⁹ PFU/mL** | |
|---|---|---|---|---|
| | **Kills** | **Unique Kills** | **Kills** | **Unique Kills** |
| ECML-123-2 | 55 | 55 | 113 | 113 |
| ECML-359 | 50 | 32 | 101 | 25 |
| CLB_P2 | 43 | 23 | 143 | 44 |
| ECML-119 | 38 | 13 | 72 | 4 |
| LF82_P8 | 37 | 13 | 111 | 12 |
| LF82_P2 | 33 | 9 | 115 | 1 |
| ECML-363 | 45 | 4 | 113 | 1 |
| 7 phage cocktail | 149 (71%) | | 200 (95%) | |

For the experiments reported herein, the seven-phage cocktail was prepared by first propagating each component bacteriophage separately in its respective *E. coli* host strain at 37°C, with Multiplicity of Infections (MOIs) ranging from 2 x 10⁻⁴ to 1 x 10⁻¹. Following propagation, each phage was harvested by filtering through a 0.2-micron filter and concentrated/buffer exchanged in a 0.9% saline solution. The seven phages were then combined at approximately equal concentrations to produce the phage cocktail, which was stored at 2-8°C until use.

Additionally, to assess the specificity of the phages, activity of the seven phage cocktail (prepared with approximately equal amounts of each phage) against a panel of 43 non-*E*. *coli* isolates was assessed, using the twelve most common genera typically associated with a healthy human microbiome: *Bacteroides* (11), *Bifidobacterium* (7), *Clostridium* (1), *Enterococcus* (9), *Fusobacterium* (1), *Lactobacillus* (1), *Parabacteroides* (1), *Prevotella* (2), *Pseudomonas* (1), *Ruminococcus* (5), *Streptococcus* (2), and *Streptomyces* (2). These non-*E*. *coli* strains are representative of a "core microbiome" of species based on studies comparing diverse healthy individuals. Notably, ciprofloxacin, which is commonly prescribed for CD patients, targeted 58% of the non-*E*. *coli* strains tested. Each isolate was screened against 2 x 10⁴ PFU/mL or 1 x 10⁹ PFU/mL of the seven-phage cocktail, and also was screened against seven of the most commonly prescribed antibiotics. None of the 43 non-*E*. *coli* strains in the panel was lysed by the seven-phage cocktail at either concentration, while the antibiotics indiscriminately lysed 16-84% of the isolates tested.

Collectively, the results show that the seven phages described herein are individually and collectively effective against a number of different AIEC strains, while at the same time being specific for *E. coli* and not lysing common bacteria of the healthy human microbiome. Thus, in contrast to the indiscriminate activity of antibiotics, the phages described herein can be used to target AIEC while having a minimal, if any, effect on the healthy human microbiome..

### EXAMPLE 4A: SAFETY-IN VIVO TESTING

The impact of a seven phage cocktail as described herein (a cocktail of phages ECML-123-2, ECML-119, ECML-359, ECML-363, CLB_P2, P2, and P8 in approximately equal amounts) on the normal microbiota of healthy BALB/cYJ mice was assessed. In particular, metagenomic analysis was performed on the stools of healthy mice (i) before, (ii) at the end of a 15-day treatment period and (iii) after a 28 day wash-out period, with treatment with the bacteriophages (2 x 10⁹ CFU/dose, b.i.d.) or placebo (n-10 per group). The experiment was performed without any antimicrobial pressure or dextran sulfate (DSS) exposure that could disrupt bacterial community structure profiles and complicate interpretation of the results.

The results showed that, apart from routine fluctuations, the dynamic composition of the gut microbiota of the phage-treated mice was not significantly different from that of the placebo group, highlighting the target specificity of the phages. Notably, administration of the bacteriophages to the healthy, unchallenged mice did not induce any clinical alterations (*i.e.,* weight loss or diarrhea) in the treated group as compared to the placebo group. These results confirmed the safety of these lytic phages and indicate that long term administration of the bacteriophages does not affect the overall fecal microbiota architecture of healthy individuals.

### EXAMPLE 4B: PHAGE ACTIVITY-IN VIVO TESTING

The ability of a bacteriophage cocktail as described herein (a cocktail of phages ECML-123-2, ECML-119, ECML-359, ECML-363, CLB_P2, P2, and P8 in approximately equal amounts) to target AIEC was assessed in an *in vivo* animal model as follows.

AIEC strain LF82 was engineered to carry two antibiotic resistance genes conferring resistance to streptomycin and kanamycin, respectively. This bacterial strain was named LF82SK and its invasive properties were verified to be similar to strain LF82.

28 BALB/cYJ mice were dispatched into three (3) groups as follows:
Group 1: LF82SK-colonized mice (10 mice)
Group 2: LF82SK-colonized mice + phage cocktail (single dose) (12 mice)
Group 3: LF82SK-colonized mice + phage cocktail (b.i.d. 15 days) (6 mice)

Streptomycin sulfate (5g/L) was introduced into the drinking water of all mice 3 days before Day 0 through Day 3. At Day 0, LF82SK was administered to the mice by oral gavage in PBS in order to allow the strain to colonize in the gut. Dextran sulfate (DSS) 2% and streptomycin (0.5mg/mL) were introduced in the drinking water on day 0 and maintained for 10 and 22 days, respectively.

At Day 8 (1) 0.9% saline was administered once to each mouse of Group 1 by oral gavage; (2) the phage cocktail (2x10⁷ PFU/mL each phage) was administered once to each mouse of Group 2 by oral gavage in 0.9% saline; and (3) the phage cocktail (2.86x10⁸ PFU/mL each phage) was administered to each mouse of Group 3 by oral gavage in 0.9% saline. For Group 3 only, this dosing was continued twice a day (b.i.d.) for 15 days (through Day 22). Weight and disease activity index (DAI) were monitored daily. At Day 24, mice from Groups 1, 2, and 3 were sacrificed to evaluate the number of bacteria in the ileum, in the colon, and in the peripheral organs, to measure colon weight and length, and to perform histological evaluations. Colons were surgically removed and subjected to length and weight measurements. Histological evaluation of samples fixed in 4% paraformaldehyde, embedded in paraffin, cut into 4 µm slices, and stained with May-Grunwald-Giemsa were graded for severity of colitis. Results are reported below, where reported values represent the mean values ± standard deviation obtained for each group of mice.

### Detection of LF82SK in feces and organs

Both LF82SK colonization and phage presence were monitored through fecal sampling. As shown in the table below, before treatment, bacterial load was similar across the three groups, reaching ca. 9 log CFU/g of feces. During phage treatment, LF82SK abundance in feces decreased in the two treatment groups, showing a significant reduction of 1.46 and 1.76 log CFU/g for Group 2 (P= 0.014) and Group 3 (P=0.005), respectively, as compared to Group 1. At Day 24, spontaneous excretion of LF82SK was comparable in all groups without significant difference.

| **LF82SK in stool (log CFU/g)** | **Group 1 (Control)** | **Group 2** | **Group 3** |
|---|---|---|---|
| | | Mean (+/- SD) | Mean (+/- SD) |
| | Mean (+/- SD) | | |
| **Day 3** | 8.91 (0.38) | 8.97 (0.4) | 9.06 (0.49) |
| **Day 8** | 7.39 (0.34) | 6.84 (0.69) NS | 6.66 (0.41)** |
| **Day 10** | 8.4 (0.69) | 6.94 (0.39) * | 6.64 (0.28)** |
| **Day 24** | 6.31 (0.64) | 6.04 (0.77) | 6.47 (0.45) |

| | | | |
|---|---|---|---|
| * P< 0.02 **P<0.01 | | | |

Phage quantification confirmed higher release in feces in Group 3 at Day 8 (6.51 ± 1.37 log PFU/g) compared to Group 1 (4.76 ± 1.04 log PFU/g). However, assessment of phage abundance by regular sampling was inconsistent and did not allow for discrimination between groups due to the detection limit. The results showed a contained phage transit through the gut without any clinical signs of distress in mice nor translocation to peripheral organs, further supporting the safe use of the bacteriophages and cocktails described herein.

LF82SK load was quantified at distinct parts of the digestive tract of mice at necropsy (Day 24), examining both luminal and mucosal samples of the terminal ileum or median colon, as reported in the table below, Regardless of the treatment (phage or placebo), no difference in LF82SK counts in feces or luminal and adherent flora was evidenced in any segment at this timepoint.

| **LF82SK load (log CFU/g)** | **Control** | **ST** | **LT** |
|---|---|---|---|
| | Mean (+/- SD) | Mean (+/- SD) | Mean (+/- SD) |
| **Ileal mucosa** | **4.10** (1.03) | **3.65** (0.99) | **3.36** (0.72) |
| **Ileal lumen** | **4.49** (1.26) | **3.74** (0.97) | **4.49** (0.94) |
| **Colonic mucosa** | **6.31** (0.64) | **6.04** (0.78) | **6.47** (0.45) |
| **Colonic lumen** | **4.76** (0.94) | **4.18** (0.53) | **4.48** (0.60) |

At day 24, (1) LF82SK (the AIEC strain) was detected in the kidneys, mesenteric lymph nodes (MLN), spleen, and heart of two of the five surviving mice of Group 1; (2) LF82SK was detected in only the MLN of one of the five surviving mice in Group 2; and (3) no LF82SK was detected in the MLNs, blood, spleen, liver, kidneys, or heart of any of the six surviving mice in Group 3.

### Survival and weight gain of mice

At day 24, 5/10 mice in Group 1 had died, 7/12 mice in Group 2 had died, and 0/6 mice in Group 3 had died. Thus, while Groups 1 and 2 were not clearly distinguished by mortality rates, the Group 3 phage treatment showed a clear effect as compared to control Group 1 (50% mortality in Group 1 vs. zero mortality in Group 3).

At day 12, Group 1 and 2 mice started to lose weight. At day 15, Group 1 and 2 reached the maximum weight loss. At day 21, Group 1 mice had recovered to their starting weight. Group 3 mice maintained or gained weight from Day 0 through Day 24. At Day 24, Group 1 mice were at approximately 97% of their starting weight (which is not unexpected, but indicates recovery of surviving animals), Group 2 mice were at approximately 108% of their starting weight, and Group 3 mice were at approximately 115% of their starting weight.

### Colonic Inflammation

At day 7, the DAI of Group 1 mice started to increase to a maximum of 4.25 (+/- 0.94) at Day 16. At day 12, the DAI of Group 2 mice started to increase to a maximum of 3.45 (+/- 1.97) at Day 15. The DAI of Group 3 mice never exceeded 0.7 +/- 0.5. The DAI of Group 3 mice were mostly due to a slightly soft consistency of stools between Day 14 and Day 17, while Group 1 and 2 mice showed weight loss, diarrhea, and some bleeding.

Group 2 and Group 3 mice showed a reduction in colonic inflammation as measured by DAI as compared to Group 1 mice. The DAI decreased with increasing levels of phage dosing.

At day 24, the colon weight/size ratio of Group 1, 2, and 3 mice were 41.4 ± 3.7, 31.8 ± 2.8, and 19.2 ± 1.1 respectively. Group 2 and Group 3 mice showed a reduction in colonic inflammation as measured by size and weight of the colon compared to Group 1 mice. The ratio of weight:size of the colon decreased with increasing levels of phage dosing.

At day 24, the histological scores of inflammations of Group 1, 2, and 3 mice were 8.2 ± 2.2, 6.4 ± 1.8, and 2.7 ± 0.3, respectively. Group 2 and Group 3 mice showed a reduction in colonic inflammation as measured by histology compared to Group 1 mice. The histological scores of inflammation decreased with increasing levels of phage dosing.

This example demonstrates efficacy of a phage cocktail as described herein against AIEC infection *in vivo.*

### EXAMPLE 5: PRODUCTION OF PHAGE PREPARATIONS

The AIEC bacteriophages described herein can be identified and propagated as illustrated herein.

For identification, nutrient broth cultures of target bacteria (*e.g*., AIEC) and putative bacteriophage preparations are mixed and then added to, for example, molten agar in nutrient broth, and that mixture is poured into a petri dish containing nutrient broth solidified with agar, and incubated overnight at 37°C. During overnight incubation, AIEC grow in the agar and form a confluent lawn with some AIEC cells being infected with bacteriophage. The phages replicate and lyse the initially infected cells and subsequently infect and lyse neighboring bacteria. The agar limits the physical spread of the phage throughout the plate, resulting in small, visibly clear areas on the plate called plaques, where bacteriophage has destroyed AIEC within the confluent lawn of AIEC growth. One plaque with a distinct morphology represents one phage that replicated in AIEC in that area of the bacterial lawn. Thus, a pure bacteriophage preparation can be obtained by removing the material in that plaque with a pipette (referred to as a "plaque pick") and using this material as the inoculum for further growth cycles of that phage. Following this approach, plaques can be harvested, diluted, and re-plated on bacterial lawns through a process of serial enrichment until a single bacteriophage species (monophage) is obtained, as may be determined by stable DNA RFLP profile.

For propagation, strains of the target bacteria (*e.g*., AIEC) or other closely related bacterial species on which the bacteriophage can propagate are cultured in batch culture using a suitable growth medium (*e.g*., BHI broth), and inoculated with the bacteriophage at a pre-determined multiplicity of infection (MOI). Following incubation and bacterial lysis, the bacteriophage is harvested and purified and/or concentrated to yield phage progeny suitable for the uses described herein.

Suitable purification and concentration procedures include one or more of filtration, centrifugation (including continuous-flow centrifugation), size-exclusion chromatography, ion exchange chromatography, and other known bacteriophage purification and concentration techniques. *See, e.g.,* Adams MH. Bacteriophages. 443-519 (Interscience Publishers, Ltd, London) (1959). The purity of the phage preparation can be assessed by one or more of electron microscopy, SDS-PAGE, DNA restriction digest, and analytical ultracentrifugation.

The bacteriophage concentration of a preparation can be adjusted using phage titration protocols. For example, the bacteriophage concentration of a preparation is determined. If a more concentrated phage preparation is desired, concentration is increased by one or more of filtration, centrifugation, or other means. If a less concentrated phage preparation is desired, the concentration is reduced by dilution with water or other pharmaceutically acceptable diluent (such as a pharmaceutically acceptable buffer). Typical pharmaceutical or OTC compositions include a phage titer of 10⁶ to 10¹² PFU/mL, such as a phage titer of 10⁹ to 10¹¹ PFU/mL.

Bacteriophage preparations may be stored at 2-8°C. Alternatively, phage preparations can be freeze-dried or spray-dried for storage, or can be encapsulated and stabilized by approaches known in the art, such as with one or more of a protein, lipid, and polysaccharide. Upon reconstitution, the phage titer can be verified using phage titration protocols, host bacteria assays, or other widely known bacteriophage assay techniques. *See, e.g.,* Adams, *supra.*

### EXAMPLE 6: PHASE I/IIa CLINICAL TRIAL

A Phase I/IIa double-blind, randomized, placebo-controlled clinical trial is underway to assess the safety and tolerability of bacteriophage cocktail as described herein (a cocktail of phages ECML-123-2, ECML-119, ECML-359, ECML-363, CLB_P2, P2, and P8), and its impact on fecal AIEC levels in subjects with non-active CD.

Eligible subjects are male or female ≥ 18 years of age with inactive Crohn's disease in clinical and objective remission with a Harvey-Bradshaw Index (HBI) < 4, and AEIC detected in the stool. Subjects with UC, colorectal tumors, gastrointestinal bleeding, active malignancies, or recent malignant disease are excluded.

Subjects will be randomized to phage treatment or placebo. The phage cocktail will be administered orally twice a day for 15 days, at least 1 hour before a meal. Each dose of the cocktail contains about 1 x 10¹⁰ PFU phages in 1 mL of a 0.9% sodium chloride solution. (The placebo is 1 mL of 0.9% sodium chloride solution.) Just prior to administration, the dose is mixed with 30mL bicarbonate water for consumption.

One week prior to starting treatment, subjects will begin taking a proton pump inhibitor (omeprazole) at a dose of 20mg daily, 30 minutes before the subject's first meal each day, to reduce stomach acid. The proton pump inhibitor treatment will be continued throughout the treatment period. Additionally, a few minutes prior to consumption of each dose, the subject will consume 120mL of bicarbonate water to neutralize stomach acid.

The primary study endpoint is the incidence and severity of treatment emergent adverse events and serious adverse events. The secondary endpoints include the level of AIEC present in stool, including all AIEC and AIEC susceptible to the phage

## Claims

1. A composition comprising a bacteriophage strain capable of producing a lytic infection in an adherent-invasive *Escherichia coli* strain, and a pharmaceutically acceptable carrier, wherein the bacteriophage strain is strain ECML-123-2, or a variant strain thereof, wherein the variant strain has at least 99% average nucleotide identity across its genome (gANI) to said bacteriophage strain ECML-123-2, and wherein the sequence of ECML-123-2 is set forth in SEQ ID NO: 1.

2. A composition according to claim 1, wherein the composition further comprises one or more bacteriophage strains selected from:
strain P2 deposited with the French National Collection of Microorganisms at the Institut Pasteur under Accession Number CNCM I-4695 or a variant strain thereof, wherein the variant strain has at least 80% gANI to said bacteriophage strain P2;
strain P8 deposited with the French National Collection of Microorganisms at the Institut Pasteur under Accession Number CNCM I-4700 or a variant strain thereof, wherein the variant strain has at least 80% gANI to said bacteriophage strain P8;
strain CLB_P2 deposited with the French National Collection of Microorganisms at the Institut Pasteur under Accession Number CNCM I-4675 or a variant strain thereof, wherein the variant strain has at least 80% gANI to said bacteriophage strain CLB_P2;
strain ECML-119 deposited with the ATCC under Accession Number PTA-8351 or a variant strain thereof, wherein the variant strain has at least 80% gANI to said bacteriophage strain ECML-119;
strain ECML-363 deposited with the ATCC under Accession Number PTA-121407 or a variant strain thereof, wherein the variant strain has at least 80% gANI to said bacteriophage strain ECML-363; and
strain ECML-359 deposited with the ATCC under Accession Number PTA-121406 or a variant thereof, wherein the variant has at least 80% gANI to said bacteriophage strain ECML-359.

3. A composition according to claim 2, wherein the bacteriophage strains present comprise or consist of (i), (ii) or (iii):
(i) strain ECML-123-2, or a variant strain thereof, wherein the variant strain has at least 99% gANI to said bacteriophage strain ECML-123-2;
strain P2, or a variant strain thereof, wherein the variant strain has at least 90% gANI to said bacteriophage strain P2;
strain P8, or a variant strain thereof, wherein the variant strain has at least 90% gANI to said bacteriophage strain P8;
strain CLB_P2, or a variant strain thereof, wherein the variant strain has at least 90% gANI to said bacteriophage strain CLB_P2;
strain ECML-119, or a variant strain thereof, wherein the variant strain has at least 90% gANI to said bacteriophage strain ECML-119;
strain ECML-363, or a variant strain thereof, wherein the variant strain has at least 90% gANI to said bacteriophage strain ECML-363; and
strain ECML-359, or a variant thereof, wherein the variant has at least 90% gANI to said bacteriophage strain ECML-359;
(ii) strain ECML-123-2, or a variant strain thereof, wherein the variant strain has at least 99% gANI to said bacteriophage strain ECML-123-2;
strain P2, or a variant strain thereof, wherein the variant strain has at least 95% gANI to said bacteriophage strain P2;
strain P8, or a variant strain thereof, wherein the variant strain has at least 95% gANI to said bacteriophage strain P8;
strain CLB_P2, or a variant strain thereof, wherein the variant strain has at least 95% gANI to said bacteriophage strain CLB_P2;
strain ECML-119, or a variant strain thereof, wherein the variant strain has at least 95% gANI to said bacteriophage strain ECML-119;
strain ECML-363, or a variant strain thereof, wherein the variant strain has at least 95% gANI to said bacteriophage strain ECML-363; and
strain ECML-359, or a variant thereof, wherein the variant has at least 95% gANI to said bacteriophage strain ECML-359;
(iii) bacteriophages of each of strains ECML-123-2, P2, P8, CLB_P2, ECML-119, ECML-363, and ECML-359.

4. A composition according to any one of the preceding claims, wherein the composition further comprises one or more of a probiotic bacteria and a probiotic yeast; optionally wherein:
(i) the composition comprises probiotic bacteria selected from one or more of *L*. *acidophilus, L. rhamnosus, L. gasseri, L. reuteri, L. bulgaricus, L. plantarum, L. johnsonii, L. paracasei, L. casei, L. salivarius, L. lactis, B. bifidum, B. longum, B. breve, B. infantis, B. lactis, B. adolescentis, Streptococcus thermophilus, Bacillus cerus, Bacillus subtilis,* and any combinations thereof; and/or
(ii) the composition comprises probiotic yeast selected from one or more of *Saccharomyces cerevisiae, Saccharomyces boulardii, Saccharomyces cerevisiae var. boulardii, Issatchenkia occidentalis, Lachancea thermotolerans, Metschnikowia ziziphicola, Torulaspora delbrueckii,* and any combination thereof.

5. A composition according to any one of the preceding claims, wherein the composition is provided in an oral dosage form, optionally provided with an enteric coating, optionally in a form selected from tablets, hard gel capsules, soft gel capsules, dragees, powders, granules, solutions, suspensions, dispersions, syrups, and microgels; optionally further comprising a gastric acid reducing agent, optionally selected from a gastric acid neutralizing agent and a proton pump inhibitor.

6. A composition according to any one of claims 1-4, wherein the composition is provided in a dosage form selected from:
a rectal dosage form, optionally in a suppository, enema, rectal foam, lotion, or gel; a vaginal dosage form, optionally in a suppository, cream vaginal foam, lotion, or gel; a pulmonary dosage form, optionally in a powder, aerosol, nebulizer, or insufflator composition; and
an injectable dosage form.

7. A composition according to any one of the preceding claims, wherein the composition is a pharmaceutical composition or an over-the-counter composition; and/or wherein the composition is a dietary supplement, nutraceutical composition, nutritional supplement, or probiotic composition.

8. A bacteriophage composition according to any one of claims 1-7, for use in reducing the risk of, or preventing, or treating adherent-invasive *Escherichia coli* (AIEC) infection in a subject in need thereof.

9. The composition for use of claim 8, wherein the subject is at risk of infection by AIEC or is infected by AIEC.

10. A bacteriophage composition according to any one of claims 1-7, for use in treating inflammatory bowel disease (IBD) in a subject in need thereof, wherein the subject is infected by AIEC.

11. The composition for use of any one of claims 8-10,
(i) wherein the AIEC is present in one or more of the small intestine or large intestine of the subject; or
(ii) wherein the AIEC is present in the colon of the subject; or
(iii) wherein the AIEC is present in the urinary tract of the subject; or
(iv) wherein the AIEC is present in the lungs the subject.

12. The composition for use of any one of claims 8 to 11,
(i) wherein the AIEC is of one or more strains selected from LF82, 07081, 07082, 07076 and 06075, optionally wherein the AIEC is or comprises strain LF82; and/or
(ii) wherein the use is effective to reduce or prevent AIEC colonization in one or more of the small intestine, large intestine, colon, urinary tract, brain and lungs of the subject; and/or
(iii) wherein the subject is suffering from an inflammatory bowel disease (IBD), optionally wherein the subject is suffering from Crohn's disease (CD), or ulcerative colitis (UC), or recurrence of ileal lesions after surgery, optionally after surgery to remove at least a part of the small intestine in a CD patient.

13. The composition for use of any one of claims 8-12,
a) wherein the bacteriophage composition is administered:
(i) orally; optionally further comprising orally administering to the subject a gastric acid reducing agent, optionally selected from a gastric acid neutralizing agent and a proton pump inhibitor; or
(ii) by a route selected from rectally, vaginally, pulmonary, and injection; and/or
b) wherein the use further comprises administering a probiotic to the subject, optionally wherein the probiotic is provided in the same composition as the bacteriophage, optionally wherein the composition is administered orally; and/or
c) wherein the subject is additionally treated with fecal microbiota transplantation.

14. A composition according to any one of claims 1-7, or the composition for use of any one of claims 8-13, wherein the variant strain of the bacteriophage strain ECML-123-2 has at least 99.9% gANI to said bacteriophage strain ECML-123-2.

15. An isolated bacteriophage of strain ECML-123-2, wherein the sequence of ECML-123-2 is set forth in SEQ ID NO: 1.

16. An isolated variant strain of the bacteriophage of claim 15, wherein the variant strain has a gANI of at least 99% or at least 99.9% to said bacteriophage strain ECML-123-2, optionally wherein the variant strain is a progeny of said bacteriophage strain ECML-123-2.

## Patentansprüche

1. Zusammensetzung, umfassend einen Bakteriophagenstamm, der fähig ist eine lytische Infektion in einem adhärenten-invasiven *Escherichia coli*-Stamm zu erzeugen, und einen pharmazeutisch verträglichen Träger, wobei der Bakteriophagenstamm der Stamm ECML-123-2 oder ein Variantenstamm davon ist, wobei der Variantenstamm mindestens 99 % durchschnittliche Nukleotididentität über sein Genom ("average nucleotide identity across its genome", gANI) mit dem Bakteriophagenstamm ECML-123-2 aufweist und wobei die Sequenz von ECML-123-2 in SEQ ID NO: 1 aufgeführt ist.

2. Zusammensetzung gemäß Anspruch 1, wobei die Zusammensetzung ferner einen oder mehrere Bakteriophagenstämme umfasst, ausgewählt aus:
Stamm P2, hinterlegt bei der French National Collection of Microorganisms am Institut Pasteur unter der Hinterlegungsnummer CNCM I-4695, oder einem Variantenstamm davon, wobei der Variantenstamm mindestens 80 % gANI mit dem genannten Bakteriophagenstamm P2 aufweist;
Stamm P8, hinterlegt bei der French National Collection of Microorganisms am Institut Pasteur unter der Hinterlegungsnummer CNCM I-4700, oder einen Variantenstamm davon, wobei der Variantenstamm mindestens 80 % gANI mit dem Bakteriophagenstamm P8 aufweist;
Stamm CLB_P2, hinterlegt bei French National Collection of Microorganisms am Institut Pasteur unter der Hinterlegungsnummer CNCM I-4675, oder ein Variantenstamm davon, wobei der Variantenstamm mindestens 80 % gANI mit dem Bakteriophagenstamm CLB_P2 aufweist;
Stamm ECML-119, hinterlegt bei der ATCC unter der Hinterlegungsnummer PTA-8351, oder ein Variantenstamm davon, wobei der Variantenstamm mindestens 80 % gANI mit dem Bakteriophagenstamm ECML-119 aufweist;
Stamm ECML-363, hinterlegt bei der ATCC unter der Hinterlegungsnummer PTA-121407, oder ein Variantenstamm davon, wobei der Variantenstamm mindestens 80 % gANI mit dem Bakteriophagenstamm ECML-363 aufweist; und
Stamm ECML-359, hinterlegt bei der ATCC unter der Hinterlegungsnummer PTA-121406, oder eine Variante davon, wobei die Variante mindestens 80 % gANI mit dem Bakteriophagenstamm ECML-359 aufweist.

3. Zusammensetzung gemäß Anspruch 2, wobei die vorhandenen Bakteriophagenstämme (i), (ii) oder (iii) umfassen oder daraus bestehen:
(i)Stamm ECML-123-2 oder einen Variantenstamm davon, wobei der Variantenstamm mindestens 99 % gANI mit dem Bakteriophagenstamm ECML-123-2 aufweist;
Stamm P2 oder einen Variantenstamm davon, wobei der Variantenstamm mindestens 90 % gANI mit dem Bakteriophagenstamm P2 aufweist;
Stamm P8 oder einen Variantenstamm davon, wobei der Variantenstamm mindestens 90 % gANI mit dem Bakteriophagenstamm P8 aufweist;
Stamm CLB_P2 oder ein Variantenstamm davon, wobei der Variantenstamm mindestens 90 % gANI mit dem Bakteriophagestamm CLB_P2 aufweist;
Stamm ECML-119 oder ein Variantenstamm davon, wobei der Variantenstamm mindestens 90 % gANI mit dem Bakteriophagestamm ECML-119 aufweist;
Stamm ECML-363 oder ein Variantenstamm davon, wobei der Variantenstamm mindestens 90 % gANI mit dem Bakteriophagenstamm ECML-363 aufweist; und
Stamm ECML-359 oder eine Variante davon, wobei die Variante mindestens 90 % gANI mit dem Bakteriophagenstamm ECML-359 aufweist;
(ii) Stamm ECML-123-2 oder einen Variantenstamm davon, wobei der Variantenstamm mindestens 99 % gANI mit dem Bakteriophagenstamm ECML-123-2 aufweist;
Stamm P2 oder einen Variantenstamm davon, wobei der Variantenstamm mindestens 95 % gANI mit dem Bakteriophagenstamm P2 aufweist;
Stamm P8 oder einen Variantenstamm davon, wobei der Variantenstamm mindestens 95 % gANI mit dem Bakteriophagenstamm P8 aufweist;
Stamm CLB_P2 oder ein Variantenstamm davon, wobei der Variantenstamm mindestens 95 % gANI mit dem Bakteriophagenstamm CLB_P2 aufweist;
Stamm ECML-119 oder ein Variantenstamm davon, wobei der Variantenstamm mindestens 95 % gANI mit dem Bakteriophagenstamm ECML-119 aufweist;
Stamm ECML-363 oder ein Variantenstamm davon, wobei der Variantenstamm mindestens 95 % gANI mit dem Bakteriophagenstamm ECML-363 aufweist; und
Stamm ECML-359 oder eine Variante davon, wobei die Variante mindestens 95 % gANI mit dem Bakteriophagenstamm ECML-359 aufweist;
(iii) Bakteriophagen von jedem der Stämme ECML-123-2, P2, P8, CLB_P2, ECML-119, ECML-363 und ECML-359.

4. Zusammensetzung gemäß irgendeinem der vorstehenden Ansprüche, wobei die Zusammensetzung ferner ein oder mehrere aus einem probiotischen Bakterium und einer probiotischen Hefe umfasst; wobei optional:
(i) die Zusammensetzung probiotische Bakterien umfasst, ausgewählt aus einem oder mehreren von *L. acidophilus, L. rhamnosus, L. gasseri, L. reuteri, L. bulgaricus, L. plantarum, L. johnsonii, L. paracasei, L. casei, L. salivarius, L. lactis, B. bifidum, B. longum, B. breve, B. infantis, B. lactis, B. adolescentis, Streptococcus thermophilus*, *Bacillus cereus, Bacillus subtilis* sowie jede Kombination davon; und/oder
(ii) die Zusammensetzung probiotische Hefe umfasst, ausgewählt aus einer oder mehreren von *Saccharomyces cerevisiae, Saccharomyces boulardii, Saccharomyces cerevisiae var. boulardii, Issatchenkia occidentalis, Lachancea thermotolerans, Metschnikowia ziziphicola, Torulaspora delbrueckii* sowie jede Kombination davon.

5. Zusammensetzung gemäß irgendeinem der vorstehenden Ansprüche, wobei die Zusammensetzung in einer oralen Darreichungsform vorliegt, optional mit einer magensaftresistenten Beschichtung versehen ist, optional in einer Form, die aus Tabletten, Hartgelkapseln, Weichgelkapseln, Dragees, Pulvern, Granulaten, Lösungen, Suspensionen, Dispersionen, Sirupen und Mikrogelen ausgewählt ist;
optional ferner umfassend ein Mittel zur Senkung der Magensäure, optional ausgewählt aus einem Mittel zur Neutralisierung der Magensäure und einem Protonenpumpenhemmer.

6. Zusammensetzung gemäß irgendeinem der Ansprüche 1-4, wobei die Zusammensetzung in einer Darreichungsform vorliegt, ausgewählt aus:
einer rektalen Darreichungsform, optional in einem Zäpfchen, Einlauf, rektalen Schaum, einer Lotion oder
einem Gel; einer vaginalen Darreichungsform, optional in einem Zäpfchen, einer Creme, einem vaginalen Schaum,
einer Lotion oder einem Gel; einer pulmonalen Darreichungsform, optional in einem Pulver, Aerosol, Vernebler oder einer Insufflator-Zusammensetzung; und
einer injizierbaren Darreichungsform.

7. Zusammensetzung gemäß irgendeinem der vorstehenden Ansprüche, wobei die Zusammensetzung eine pharmazeutische Zusammensetzung oder eine rezeptfreie Zusammensetzung ist; und/oder wobei die Zusammensetzung ein Nahrungsergänzungsmittel, eine nutrazeutische Zusammensetzung, ein Nahrungszusatz oder eine probiotische Zusammensetzung ist.

8. Bakteriophagen-Zusammensetzung gemäß irgendeinem der Ansprüche 1-7 zur Verwendung bei der Verringerung des Risikos oder der Vorbeugung oder der Behandlung einer Infektion mit adhärent-invasiven *Escherichia coli* (AIEC) bei einem Subjekt, das dies benötigt.

9. Zusammensetzung zur Verwendung nach Anspruch 8, wobei der Patient gefährdet ist, eine Infektion mit AIEC zu bekommen, oder mit AIEC infiziert ist.

10. Bakteriophagen-Zusammensetzung gemäß irgendeinem der Ansprüche 1-7 zur Verwendung bei der Behandlung einer entzündlichen Darmerkrankung ("inflammatory bowel disease", IBD) bei einem Subjekt, das dies benötigt, wobei das Subjekt mit AIEC infiziert ist.

11. Zusammensetzung zur Verwendung gemäß irgendeinem der Ansprüche 8-10,
(i) wobei die AIEC in einem oder mehreren von Dünndarm oder Dickdarm des Subjekts vorhanden ist; oder
(ii) wobei die AIEC im Dickdarm des Subjekts vorhanden ist; oder
(iii) wobei die AIEC im Harntrakt des Subjekts vorhanden ist; oder
(iv) wobei die AIEC in der Lunge des Subjekts vorhanden ist.

12. Zusammensetzung zur Verwendung gemäß irgendeinem der Ansprüche 8 bis 11,
(i) wobei das AIEC aus einem oder mehreren Stämmen besteht, ausgewählt aus LF82, 07081, 07082, 07076 und 06075, wobei das AIEC optional der Stamm LF82 ist, oder diesen umfasst; und/oder
(ii) wobei die Verwendung wirksam ist, um die Besiedlung durch AIEC in einem oder mehreren von Dünndarm, Dickdarm, Kolon, Harnwegen, Gehirn und Lunge des Subjekts zu verringern oder zu verhindern; und/oder
(iii) wobei das Subjekt an einer entzündlichen Darmerkrankung (IBD) leidet, wobei das Subjekt gegebenenfalls an Morbus Crohn ("Crohn's disease", CD) oder Colitis ulcerosa ("ulcerative colitis", UC) oder an einem Wiederauftreten von Ileumläsionen nach einer Operation leidet, optional nach einer Operation zur Entfernung zumindest eines Teils des Dünndarms bei einem CD-Patienten.

13. Zusammensetzung zur Verwendung gemäß irgendeinem der Ansprüche 8 bis 12,
a) wobei die Bakteriophagen-Zusammensetzung verabreicht wird:
(i) oral; optional ferner umfassend das orale Verabreichen eines Mittels zur Senkung der Magensäure an das Subjekt, das optional aus einem Mittel zur Neutralisierung der Magensäure und einem Protonenpumpenhemmer ausgewählt ist; oder
(ii) auf einem Verabreichungsweg, ausgewählt aus rektal, vaginal, pulmonal und Injektion; und/oder
b) wobei die Verwendung ferner das Verabreichen eines Probiotikums an das Subjekt umfasst, wobei das Probiotikum optional in derselben Zusammensetzung wie der Bakteriophage bereitgestellt wird, wobei die Zusammensetzung optional oral verabreicht wird; und/oder
c) wobei das Subjekt zusätzlich mit einer Stuhltransplantation behandelt wird.

14. Zusammensetzung gemäß irgendeinem der Ansprüche 1-7 oder die Zusammensetzung zur Verwendung gemäß irgendeinem der Ansprüche 8-13, wobei der Variantenstamm des Bakteriophagenstamms ECML-123-2 mindestens 99,9 % gANI mit dem genannten Bakteriophagenstamm ECML-123-2 aufweist.

15. Isolierter Bakteriophage des Stammes ECML-123-2, wobei die Sequenz von ECML-123-2 in SEQ ID NO: 1 aufgeführt ist.

16. Isolierter Variantenstamm des Bakteriophagen nach Anspruch 15, wobei der Variantenstamm eine gANI von mindestens 99 % oder mindestens 99,9 % mit dem Bakteriophagenstamm ECML-123-2 aufweist, wobei der Variantenstamm optional ein Nachkomme des Bakteriophagenstamms ECML-123-2 ist.

## Revendications

1. Composition comprenant une souche de bactériophage apte à produire une infection lytique dans une souche d'*Escherichia coli* adhérente et invasive, et un vecteur pharmaceutiquement acceptable, dans laquelle la souche de bactériophage est la souche ECML-123-2, ou une souche variante de celle-ci, dans laquelle la souche variante présente au moins 99 % d'identité nucléotidique moyenne sur l'ensemble de son génome (gANI) par rapport à ladite souche de bactériophage ECML-123-2, et dans laquelle la séquence d'ECML-123-2 est exposée dans SEQ ID NO : 1.

2. Composition selon la revendication 1, dans laquelle la composition comprend en outre une ou plusieurs souches de bactériophage sélectionnées parmi :
la souche P2 déposée auprès de la Collection Nationale de Cultures de Microorganismes de l'Institut Pasteur sous le numéro d'accès CNCM I-4695 ou une souche variante de celle-ci, dans laquelle la souche variante présente au moins 80 % de gANI par rapport à ladite souche de bactériophage P2 ;
la souche P8 déposée auprès de la Collection Nationale de Cultures de Microorganismes de l'Institut Pasteur sous le numéro d'accès CNCM I-4700 ou une souche variante de celle-ci, dans laquelle la souche variante présente au moins 80 % de gANI par rapport à ladite souche de bactériophage P8 ;
la souche CLB_P2 déposée auprès de la Collection Nationale de Cultures de Microorganismes de l'Institut Pasteur sous le numéro d'accès CNCM I-4675 ou une souche variante de celle-ci, dans laquelle la souche variante présente au moins 80 % de gANI par rapport à ladite souche de bactériophage CLB_P2 ;
la souche ECML-119 déposée auprès de l'ATCC sous le numéro d'accès PTA-8351 ou une souche variante de celle-ci, dans laquelle la souche variante présente au moins 80 % de gANI par rapport à ladite souche de bactériophage ECML-119 ;
la souche ECML-363 déposée auprès de l'ATCC sous le numéro d'accès PTA-121407 ou une souche variante de celle-ci, dans laquelle la souche variante présente au moins 80 % de gANI par rapport à ladite souche de bactériophage ECML-363 ; et
la souche ECML-359 déposée auprès de l'ATCC sous le numéro d'accès PTA-121406 ou une variante de celle-ci, dans laquelle ladite variante présente au moins 80 % de gANI par rapport à ladite souche de bactériophage ECML-359.

3. Composition selon la revendication 2, dans laquelle les souches de bactériophage présentes comprennent ou consistent en (i), (ii) ou (iii) :
(i) la souche ECML-123-2, ou une souche variante de celle-ci, dans laquelle la souche variante présente au moins 99 % de gANI par rapport à ladite souche de bactériophage ECML-123-2 ;
la souche P2, ou une souche variante de celle-ci, dans laquelle la souche variante présente au moins 90 % de gANI par rapport à ladite souche de bactériophage P2 ;
la souche P8, ou une souche variante de celle-ci, dans laquelle la souche variante présente au moins 90 % de gANI par rapport à ladite souche de bactériophage P8 ;
la souche CLB_P2, ou une souche variante de celle-ci, dans laquelle la souche variante présente au moins 90 % de gANI par rapport à ladite souche de bactériophage CLB_P2 ;
la souche ECML-119, ou une souche variante de celle-ci, dans laquelle la souche variante présente au moins 90 % de gANI par rapport à ladite souche de bactériophage ECML-119 ;
la souche ECML-363, ou une souche variante de celle-ci, dans laquelle la souche variante présente au moins 90 % de gANI par rapport à ladite souche de bactériophage ECML-363 ; et
la souche ECML-359, ou un variant de celle-ci, dans laquelle ledit variant présente au moins 90 % de gANI par rapport à ladite souche de bactériophage ECML-359 ;
(ii) la souche ECML-123-2, ou une souche variante de celle-ci, dans laquelle la souche variante présente au moins 99 % de gANI par rapport à ladite souche de bactériophage ECML-123-2 ;
la souche P2, ou une souche variante de celle-ci, dans laquelle la souche variante présente au moins 95 % de gANI par rapport à ladite souche de bactériophage P2 ;
la souche P8, ou une souche variante de celle-ci, dans laquelle la souche variante présente au moins 95 % de gANI par rapport à ladite souche de bactériophage P8 ;
la souche CLB_P2, ou une souche variante de celle-ci, dans laquelle la souche variante présente au moins 95 % de gANI par rapport à ladite souche de bactériophage CLB_P2 ;
la souche ECML-119, ou une souche variante de celle-ci, dans laquelle la souche variante présente au moins 95 % de gANI par rapport à ladite souche de bactériophage ECML-119 ;
la souche ECML-363, ou une souche variante de celle-ci, dans laquelle la souche variante présente au moins 95 % de gANI par rapport à ladite souche de bactériophage ECML-363 ; et
la souche ECML-359, ou un variant de celle-ci, dans laquelle ledit variant présente au moins 95 % de gANI par rapport à ladite souche de bactériophage ECML-359 ;
(iii) les bactériophages de chacune des souches ECML-123-2, P2, P8, CLB_P2, ECML-119, ECML-363 et ECML-359.

4. Composition selon l'une quelconque des revendications précédentes, dans laquelle la composition comprend en outre une ou plusieurs parmi une bactérie probiotique et/ou une levure probiotique ; facultativement, dans laquelle :
(i) la composition comprend une bactérie probiotique sélectionnée parmi un ou plusieurs de *L. acidophilus, L. rhamnosus, L. gasseri, L. reuteri, L. bulgaricus, L. plantarum, L. johnsonii, L. paracasei, L. casei, L. salivarius, L. lactis, B. bifidum, B. longum, B. breve, B. infantis, B. lactis, B. adolescentis, Streptococcus thermophilus, Bacillus cereus, Bacillus subtilis, et toutes combinaisons de ceux-ci ; et*/*ou*
(ii) la composition comprend une levure probiotique sélectionnée parmi un ou plusieurs de *Saccharomyces cerevisiae, Saccharomyces boulardii, Saccharomyces cerevisiae var. boulardii, Issatchenkia occidentalis, Lachancea thermotolerans, Metschnikowia ziziphicola, Torulaspora delbrueckii, et toute combinaison de ceux-ci.*

5. Composition selon l'une quelconque des revendications précédentes, dans laquelle la composition est présentée sous une forme posologique orale, facultativement pourvue d'un enrobage entérique, facultativement sous une forme sélectionnée parmi les comprimés, les capsules de gel dures, les capsules de gel molles, les dragées, les poudres, les granulés, les solutions, les suspensions, les dispersions, les sirops et les microgels ; comprenant facultativement en outre un agent réduisant l'acidité gastrique, facultativement sélectionné parmi un agent neutralisant l'acidité gastrique et un inhibiteur de la pompe à protons.

6. Composition selon l'une quelconque des revendications 1 à 4, dans laquelle la composition est présentée sous une forme posologique sélectionnée parmi :
une forme posologique rectale, facultativement sous forme de suppositoire, lavement, mousse rectale, lotion rectale ou gel rectal ; une forme posologique vaginale, facultativement sous forme de suppositoire, crème, mousse vaginale, lotion vaginale ou gel vaginal ; une forme posologique pulmonaire, facultativement sous forme de composition en poudre pour aérosol, nébuliseur ou insufflateur ; et
une forme posologique injectable.

7. Composition selon l'une quelconque des revendications précédentes, dans laquelle la composition est une composition pharmaceutique ou une composition en vente libre ; et/ou dans laquelle la composition est un complément alimentaire, une composition nutraceutique, un complément nutritionnel ou une composition probiotique.

8. Composition de bactériophage selon l'une quelconque des revendications 1 à 7, pour utilisation dans la réduction du risque d'infection par *Escherichia coli* adhérente et invasive (AIEC), ou dans la prévention ou dans le traitement de ladite infection, chez un sujet qui en a besoin.

9. Composition pour utilisation selon la revendication 8, dans laquelle le sujet présente un risque d'infection par l'AIEC ou est infecté par l'AIEC.

10. Composition de bactériophage selon l'une quelconque des revendications 1 à 7, pour utilisation dans le traitement d'une maladie inflammatoire de l'intestin (MII) chez un sujet qui en a besoin, dans laquelle le sujet est infecté par l'AIEC.

11. Composition pour utilisation selon l'une quelconque des revendications 8 à 10,
(i) dans laquelle l'AIEC est présente dans l'intestin grêle et/ou le gros intestin du sujet ; ou
(ii) dans laquelle l'AIEC est présente dans le côlon du sujet ; ou
(iii) dans laquelle l'AIEC est présente dans les voies urinaires du sujet ; ou
(iv) dans laquelle l'AIEC est présente dans les poumons du sujet.

12. Composition pour utilisation selon l'une quelconque des revendications 8 à 11,
(i) dans laquelle l'AIEC est d'une ou de plusieurs souches sélectionnées parmi LF82, 07081, 07082, 07076 et 06075, facultativement dans laquelle l'AIEC est ou comprend la souche LF82 ; et/ou
(ii) dans laquelle l'utilisation est efficace pour réduire ou prévenir la colonisation par AIEC dans un ou plusieurs parmi l'intestin grêle, le gros intestin, le côlon, les voies urinaires, le cerveau et les poumons du sujet ; et/ou
(iii) dans laquelle le sujet souffre d'une maladie inflammatoire de l'intestin (MII), facultativement dans laquelle le sujet souffre de la maladie de Crohn (MC), ou de colite ulcéreuse (CU), ou de récurrence de lésions iléales après une intervention chirurgicale, facultativement après une intervention chirurgicale visant à retirer au moins une partie de l'intestin grêle chez un patient atteint de la MC.

13. Composition pour utilisation selon l'une quelconque des revendications 8 à 12,
a) dans laquelle la composition de bactériophage est administrée :
(i) par voie orale ; comprenant facultativement en outre l'administration par voie orale, au sujet, d'un agent réduisant l'acidité gastrique, facultativement sélectionné parmi un agent neutralisant l'acidité gastrique et un inhibiteur de la pompe à protons ; ou
(ii) par une voie sélectionnée parmi la voie rectale, la voie vaginale, la voie pulmonaire et l'injection ; et/ou
b) dans laquelle l'utilisation comprend en outre l'administration d'un probiotique au sujet, facultativement dans laquelle le probiotique est présenté dans la même composition que le bactériophage, facultativement dans laquelle la composition est administrée par voie orale ; et/ou
c) dans laquelle le sujet est en plus traité par transplantation de microbiote fécal.

14. Composition selon l'une quelconque des revendications 1 à 7, ou composition pour utilisation selon l'une quelconque des revendications 8 à 13, dans laquelle la souche variante de la souche de bactériophage ECML-123-2 présente au moins 99,9 % de gANI par rapport à ladite souche de bactériophage ECML-123-2.

15. Bactériophage isolé de la souche ECML-123-2, dans lequel la séquence d'ECML-123-2 est énoncée dans SEQ ID NO : 1.

16. Souche variante isolée du bactériophage selon la revendication 15, dans laquelle la souche variante présente un gANI d'au moins 99 % ou d'au moins 99,9 % par rapport à ladite souche de bactériophage ECML-123-2, facultativement dans laquelle la souche variante est une descendance de ladite souche de bactériophage ECML-123-2.
